# FASCICULE DE BREVET EUROPEEN

(11) **EP 1 622 925 B1**
(45) Date de publication et mention de la délivrance du brevet: **09.03.2011**
(21) Numéro de dépôt: 04742577.2
(22) Date de dépôt: 27.04.2004
(51) Int. Cl.: C07J 1/00, C07J 43/00, C07J 75/00, A61K 31/566, A61K 31/58, A61P 19/10

(54) **NOUVEAU PROCEDE ET INTERMEDIAIRES DE PREPARATION DE COMPOSES 19-NOR-STEROÏDES**
NEUES VERFAHREN UND ZWISCHENPRODUKTE ZUR HERSTELLUNG VON 19-NOR-STEROIDEN
NOVEL METHOD AND INTERMEDIATES FOR THE PREPARATION OF 19-NOR-STEROID COMPOUNDS

(30) Priorité: 29.04.2003 FR 0305221
(43) Date de publication de la demande: 08.02.2006
(73) Titulaire: Aventis Pharma S.A., 92160 Antony (FR)
(72) Inventeur: PRAT, Denis, F-93500 Pantin (FR); MORATILLE, Christian, F-94360 Bry sur Marne (FR); BENEDETTI, Françoise, F-93110 Rosny sous Bois (FR); NAIT-BOUDA, Lahlou, F- 94320 Thiais (FR)
(74) Mandataire: Ulmann, Catherine Claire
(86) Numéro de dépôt international: PCT/FR2004/001010
(87) Numéro de publication internationale: WO 2004/096828

(56) Documents cités:
- WO-A-98/45316
- WO-A-99/25725
- WO-A-99/67274
- WO-A-02/100880
- FR-A- 2 640 977
- LARKIN, JOHN PATRICK ET AL: "The synthesis of 17.alpha.-methyl-11.beta.-arylestradiol: large-scale application of the cerium (III)-mediated alkylation of a ketone" ORGANIC PROCESS RESEARCH & DEVELOPMENT, vol. 6, no. 1, 2002, pages 20-27, XP002266836

## Description

La présente invention a pour objet un procédé de préparation de composés 19-nor-stéroïdes, ainsi que les composés intermédiaires préparés lors de la mise en oeuvre de ce procédé.

L'ostéoporose est une maladie de l'os qui touche 50 millions de personnes dans le monde, plus particulièrement les femmes. Son développement est lié à l'âge et commence le plus souvent après la ménopause. Cette maladie est caractérisée par une réduction de la densité osseuse, entraîne des déformations, des tassements vertébraux et à terme des fractures spontanées. L'ostéoporose représente donc un sérieux enjeu de santé publique. Le principal traitement consiste dans une prise régulière d'estrogènes qui diminue la perte osseuse, qui cependant peut s'accompagner de certains effets secondaires (saignements, bouffées de chaleur, risque de cancer mammaires ...) . Une nouvelle série de molécules appelées SERM (Selective Estrogen Receptor Modulator) permet le traitement de l'ostéoporose tout en évitant certains des effets secondaires. (WO98/45316, WO99/67274, WO98/28324, WO99/25725, EP605193, WO02/100880).

L'objet de la présente demande est la mise au point d'un nouveau procédé de préparation d'un intermédiaire clé (composé de formule I) dans la synthèse de certains dérivés estrogènes ayant une activité dissociée.

L'invention a pour objet un procédé de préparation de composés de formule générale (I): 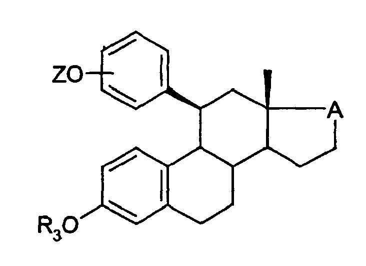 dans laquelle
Z représente un radical alkyle linéaire ou un groupement R₄ : 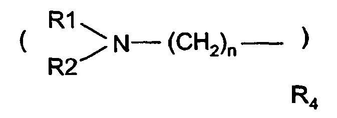 dans lequel n est un entier de 2 à 8, et
- soit R₁ et R₂, identiques ou différents représentent un groupement benzyle ou un radical alkyle, alkényle ou alkynyle linéaire, ramifié ou cyclique renfermant de 1 à 8 atomes de carbone
- soit R₁ et R₂ forment ensemble avec l'atome d'azote qui les porte un hétérocycle de 5 à 6 chaînons saturé ou insaturé, aromatique ou non aromatique renfermant éventuellement de 1 à 3 hétéroatomes additionnels et éventuellement accolé à un autre cycle,
A représente une fonction céto ou un groupement CH-X, X représentant un atome d'halogène,
R₃ représente un atome d'hydrogène ou un groupement protecteur de la fonction hydroxy,
comprenant les étapes suivantes ;
a) on soumet le mélange des composés de formules (IIIa) et (IIIb): 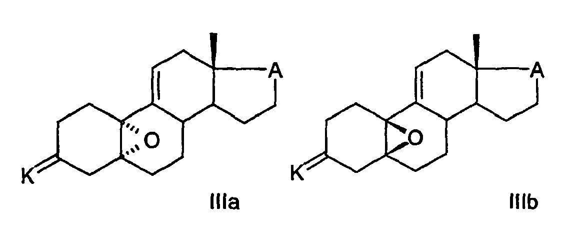 =K représentant une fonction céto protégée notamment sous forme de cétal, de thiocétal ou de cétal mixte,
   à une réaction d'alkylation avec un dérivé organocuprate dérivé d'un organométallique de formule R₅MgHal ou R₅Li, Hal étant un atome d'halogène et généré de façon catalytique ou stoechiométrique, dans laquelle R₅ représente le groupement : 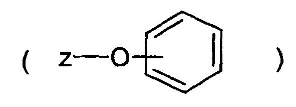 Z étant tel que défini plus haut, la liaison s'effectuant au niveau du phényle,
   puis à l'action d'un agent de déprotection afin d'obtenir les composés de formules (Va), (Vb) et (Vc) : 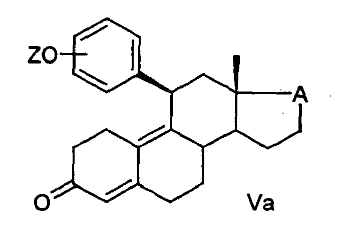 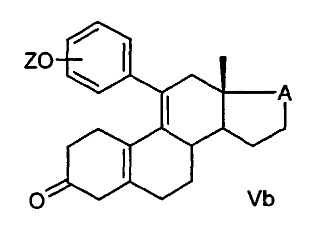 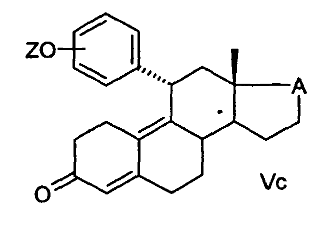
b) on traite les composés de formules (Va), (Vb) et (Vc) avec un agent d'aromatisation pour obtenir le mélange des composés de formules (VI) et (I) : 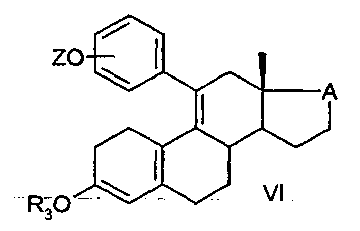 qui continuent à s'aromatiser pour obtenir le composé de formule (I), R₃ étant tel que défini plus haut.

L'invention a également pour objet un procédé de préparation de composés de formule générale (I) : 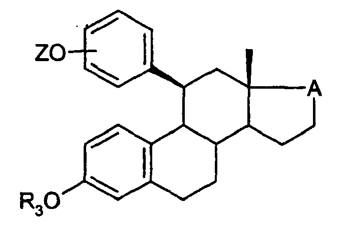 dans laquelle
Z représente un radical alkyle linéaire ou un groupement R₄ : 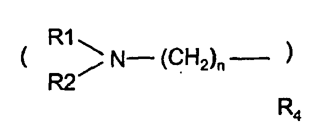 dans lequel n est un entier de 2 à 8, et
- soit R₁ et R₂, identiques ou différents représentent un groupement benzyle ou un radical alkyle, alkényle ou alkynyle linéaire, ramifié ou cyclique renfermant de 1 à 8 atomes de carbone
- soit R₁ et R₂ forment ensemble avec l'atome d'azote qui les porte un hétérocycle de 5 à 6 chaînons saturé ou insaturé, aromatique ou non aromatique renfermant éventuellement de 1 à 3 hétéroatomes additionnels et éventuellement accolé à un autre cycle,
A représente une fonction céto ou un groupement CH-X, X représentant un atome d'halogène,
R₃ représente un atome d'hydrogène ou un groupement protecteur de la fonction hydroxy,
comprenant-les étapes suivantes ;
a) on soumet le composé de formule (IIIb) : 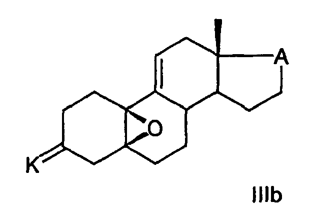 =K représentant une fonction céto protégée notamment sous forme de cétal, de thiocétal ou de cétal mixte,
   à une réaction d'alkylation avec un dérivé organocuprate dérivé d'un organométallique de formule R₅MgHal ou R₅Li, Hal étant un atome d'halogène et généré de façon catalytique ou stoechiométrique, dans laquelle R₅ représente le groupement : 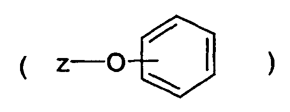 Z étant tel que défini plus haut, la liaison s'effectuant au niveau du phényle,
   puis à l'action d'un agent de déprotection afin d'obtenir les composés de formules (Vb) et (Vc) : 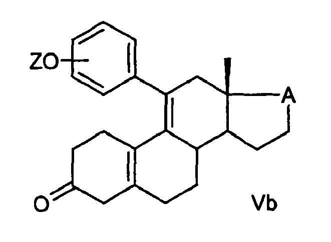 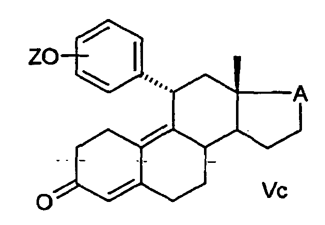
b) on traite les composés de formules (Vb) et (Vc) avec un agent d'aromatisation pour obtenir le mélange des composés de formules (VI) et (I) : 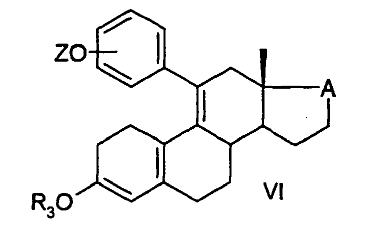 qui continuent à s'aromatiser pour obtenir le composé de formule (I), R₃ étant tel que défini plus haut.

Le groupement (ZO-) peut être en position ortho, méta, ou para.

Comme exemple de radical alkyle linaire que peut représenter Z, on peut citer les radicaux méthyle, éthyle, propyle, butyle, pentyle, hexyle, octyle. Le radical alkyle linaire que peut représenter Z est de préférence le méthyle.

Comme exemple de radical alkyle linéaire ou ramifié renfermant de 1 à 8 atomes carbones que peuvent représenter R₁ et R₂, on peut citer les radicaux méthyle, éthyle, propyle, butyle, pentyle, hexyle, octyle, et les isomères ramifiés de ces radicaux, isopropyle, isobutyle, isopentyle, neopentyle, isohexyle, 3-méthylpentyle, , sec-butyle, tert-butyle, tert-pentyle. Les radicaux alkyles préférés sont méthyle et éthyle.

Comme exemple de radical alkyle cyclique que peuvent représenter R₁ et R₂, on peut citer les radicaux cyclopropyle, cyclobutyle, cyclopentyle et cyclohexyle, cycloheptyle, ou cyclooctyle qui peuvent être substitués par exemple par un groupement alkyle renfermant de 1 à 4 atomes de carbone.

Comme exemple de radical alkényle que peuvent représenter R₁ et R₂, on peut citer les radicaux allyle, butényle ou 3-méthyl-2-butényle. Comme exemple de radicaux alkynyle on peut citer le radical propargyle. Bien entendu ces radicaux alkényle ou alkynyle renferment au moins 2 atomes de carbone et sont liés à l'atome d'azote via un groupement -CH₂-.

Comme exemple d'hétérocycle que peuvent former R₁ et R₂ ensemble avec l'atome d'azote auquel ils sont liés, on peut citer notamment des hétérocycles mono ou bicycliques renfermant éventuellement un autre hétéroatome choisi parmi l'oxygène et l'azote tels que les hétérocycles insaturés suivants : pyrrolyle, imidazolyle, indolyle, pyridyle, pyrazinyle, pyrimidinyle, pyridazinyle, thiazolyle, oxazolyle, furazolinyle, pyrazolinyle. ; ou tels que les hétérocycles saturés suivants : morpholinyle, pyrrolidinyle, piperidinyle, oxazolidinyle, thiazolidinyle. Il s'agira de préférence du groupement :

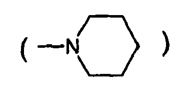

Comme exemple d'atome d'halogène que peut représenter Hal, on peut citer le chlore, l'iode ou le brome.

Comme exemple d'atome d'halogène que peut représenter X, on peut citer le chlore, le brome, l'iode, le fluor. Il s'agit de préférence du fluor.

Comme exemple de groupement protecteur que peut représenter R₃, on peut citer notamment un groupement (C₁-C₆) -alkyle, (C₁-C₆) -alkyle-CO- tel que CH₃CO ou benzoyle, benzyle, phény]-(C₁-C₆)-alkyle tel que benzyle, ainsi que tous les groupements protecteurs connus de l'homme du métier par exemple ceux décrits dans Greene, Wuts, Protective Groups in Organic Synthesis 3rd edition, Wiley & sons, 1999. De préférence R₃, à titre de groupement protecteur, est un groupement acyle.

Comme exemple de groupe protecteur du céto en position 3 du stéroide que peut représenter =K on peut citer notamment
- les cétals cycliques tels que -O-(CH₂)ₘ-O-, -O-(CH₂)ₘ-S-, -S-(CH₂)ₘ-S-. -O-CH₂-C(C₁₋₄-alkyl)₂-CH₂-O-,
- les cétals acycliques tels que (CH₃O)₂, (EtO)₂
- ainsi que tous les groupements protecteurs du groupement céto connus de l'homme du métier par exemple ceux décrits dans Greene, Wuts, Protective Groups in Organic Synthesis 3rd edition, Wiley & sons, 1999. De préférence, =K est un cétal cyclique, et notamment un groupement 3,3-éthylènedioxy.

L'invention a plus particulièrement pour objet un procédé de préparation tel que défini plus haut, de composés de formule générale (I) dans laquelle A est une fonction céto, comprenant les étapes suivantes ;
a) on soumet un composé de formule (II) : 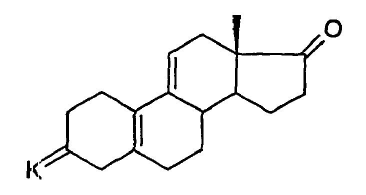 =K représentant une fonction céto protégée notamment sous forme de cétal, de thiocétal ou de cétal mixte, à l'action d'un réactif d'époxydation afin d'obtenir le mélange des isomères alpha et bêta de formules (III'a) et (III'b) : 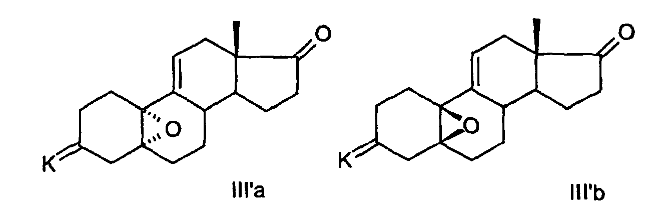
b) on soumet le mélange des composés de formules (III'a) et (III'b) à une réaction d'alkylation avec un dérivé organocuprate dérivé d'un organométallique de formule R₅MgHal ou R₅Li, Hal étant un atome d'halogène et R₅ étant tel que défini plus haut, la liaison s'effectuant au niveau du phényle,
   puis à l'action d'un agent de déprotection afin d'obtenir les composés de formules (V'a), (V'b) et (V'c) : 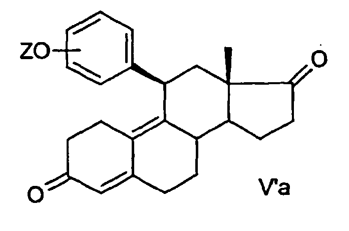 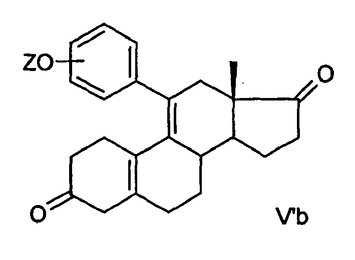 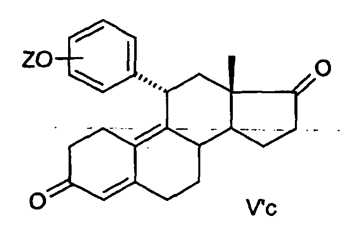
c) on traite les composés de formules (V'a), (V'b) et (V'c) avec un agent d'aromatisation pour obtenir le mélange des composés de formules (VI') et (I) dans laquelle A est une fonction céto : 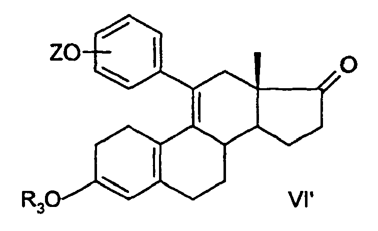 qui continuent à s'aromatiser pour obtenir le composé de formule (I) dans laquelle A est une fonction céto,
d) le cas échéant on déprotège le produit obtenu au stade c pour obtenir un composé de formule (I) dans laquelle A est une fonction céto et R₃ représente un atome d'hydrogène,
que l'on soumet le cas échéant à une réaction de salification.

L'invention a plus particulièrement pour objet un procédé de préparation tel que défini plus haut, de composés de formule générale (I) dans laquelle A est une fonction céto, comprenant les étapes suivantes ;
a) on soumet un composé de formule (II) : 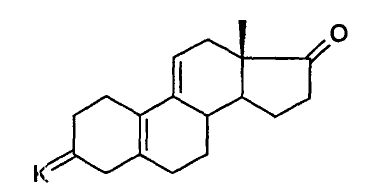 =K représentant une fonction céto protégée notamment sous forme de cétal, de thiocétal- ou de cétal mixte, à l'action d'un réactif d'époxydation afin d'obtenir l'isomère bêta de formule (III'b) : 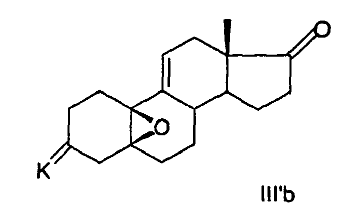
b) on soumet le composé de formule (III'b) à une réaction d'alkylation avec un dérivé organocuprate dérivé d'un organométallique de formule R₅MgHal ou R₅Li, Hal étant un atome d'halogène et R₅ étant tel que défini plus haut, la liaison s'effectuant au niveau du phényle,
   puis à l'action d'un agent de déprotection afin d'obtenir les composés de formules (V'b) et (V'c): 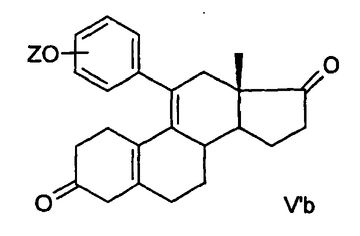 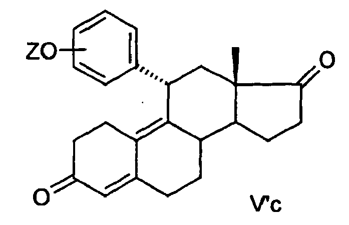
c) on traite les composés de formules (V'b) et (V'c) avec un agent d'aromatisation pour obtenir le mélange des composés de formules (VI') et (I) dans laquelle A est une fonction céto : 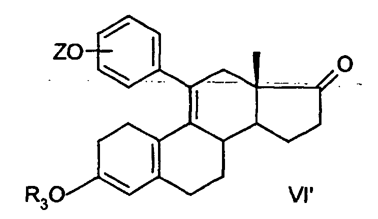 qui continuent à s'aromatiser pour obtenir le composé de formule (I) dans laquelle A est une fonction céto,
d) le cas échéant on déprotège le produit obtenu au stade c pour obtenir un composé de formule (I) dans laquelle A est une fonction céto et R₃ représente un atome d'hydrogène,
que l'on soumet le cas échéant à une réaction de salification.

La réaction d'époxydation est une réaction classique qui s'effectue selon les méthodes connues de l'homme du métier. Elle peut s'effectuer notamment en présence d'hexachloroacétone, de dichlorométhane et d'eau oxygénée.

La réaction d'alkylation avec un dérivé organocuprate dérivé d'un organométallique de formule R₅MgHal ou R₅Li, Hal et R₅ étant tels que définis plus haut, s'effectue selon les méthodes classiques connues de l'homme du métier.

La réaction de déprotection qui permet d'obtenir les composés de formules (V'a), (V'c), ou (V'b) s'effectue selon les méthodes classiques connues de l'homme du métier. L'agent de déprotection utilisé est notamment un agent permettant une hydrolyse acide, tel que l'acide chlorhydrique ou l'acide perchlorique.

La réaction d'aromatisation est une réaction classique qui s'effectue notamment selon les méthodes décrites dans EP 298,020. Cette aromatisation peut s'effectuer par catalyse avec du palladium ou de préférence en présence de bromure d'acétyle et d'anhydride acétique.

La déprotection du groupe acétyle en 3 formé le cas échéant s'effectue en général en présence d'une base forte, telle que la soude ou la potasse, dans un alcool, tel que le méthanol ou l'éthanol. Elle s'effectue de préférence en présence de soude dans le méthanol.

La réaction de salification est effectuée par les méthodes classiques connues de l'homme du métier.

L'invention a tout particulièrement pour objet un procédé tel que défini plus haut caractérisé en ce que la réaction d'alkylation s'accompagne d'une réaction d'énolisation de la fonction céto en position 17. La réaction d'énolisation s'effectue selon les conditions classiques connues de l'homme du métier. Elle s'effectue notamment par l'action d'un équivalent supplémentaire d'un réactif de Grignard.

L'invention a tout particulièrement pour objet un procédé tel que défini plus haut caractérisé en ce que l'on traite les composés de formules (III'a) et (III'b) avec un agent de silylation en présence d'une base, afin d'obtenir le mélange des énols silylés de formules (IVa) et (IVb) : 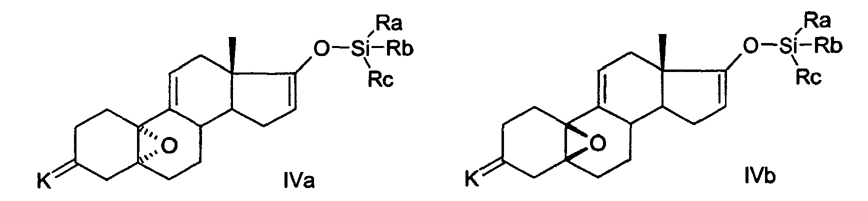 dans lesquelles Ra, Rb et Rc identiques ou différents représentent un radical alkyle renfermant de 1 à 4 atomes de carbone, ou un radical phényl,
que l'on fait réagir avec dérivé organocuprate tel que défini plus haut, la liaison s'effectuant au niveau du phényle,
afin d'obtenir les composés de formules (IV'a) et (IV'b) qui sont isolés ou non isolés : 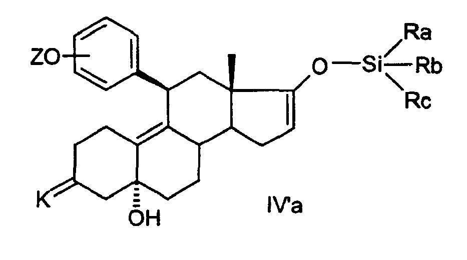 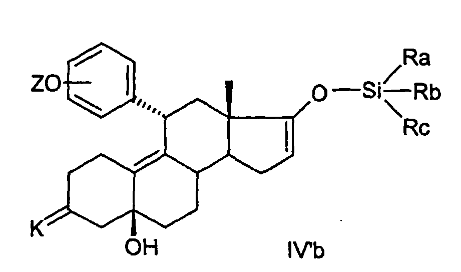 lesdits produits étant ensuite déprotégés pour obtenir les composés de formules (V'a), (V'b), (V'c) telles que définies plus haut.

L'invention a tout particulièrement pour objet un procédé tel que défini plus haut caractérisé en ce que l'on traite le composé de formule (III'b)
avec un agent de silylation en présence d'une base, afin d'obtenir l'énol silylé de formule (IVb) : 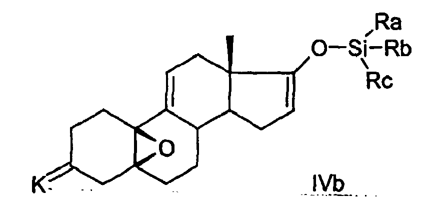 dans lesquelles Ra, Rb et Rc identiques ou différents représentent un radical alkyle renfermant de 1 à 4 atomes de carbone, ou un radical phényl,
que l'on fait réagir avec dérivé organocuprate tel que défini plus haut, la liaison s'effectuant au niveau du phényle,
afin d'obtenir le composé de formule (IV'b) qui est isolé ou non isolé : 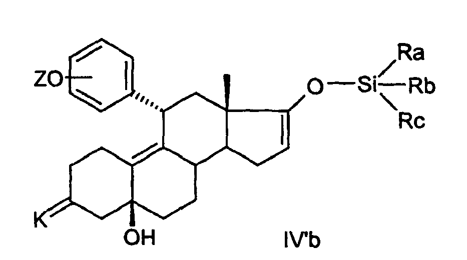 ledit produit étant ensuite déprotégé pour obtenir les composés de formules (V'b), (V'c) telles que définies plus haut.

Comme agent de silylation, on peut citer tous les agents susceptibles de silyler des énols ou des énolates connus de l'homme du métier et cités dans la monographie Van Look, G. ; Simchen, G. ; Heberle, J., Silylating Agents ; Fluka Chimica, Fluka Chemie AG ; Buchs, Switzerland, 1995. Il peut s'agir préférence de chlorosilane, tel que le triméthylchlorosilane.
Cette réaction de silylation s'effectue généralement en présence d'une base forte telle que le Li-HMDS ((Me₃Si)₂N-Li), ou la LDA ((iPr)₂N-Li).
Les solvants utilisés sont connus de l'homme du métier pour ce type de réaction. Il faut éviter les solvants protiques ou énolisables. De préférence la réaction de silylation s'effectuera avec le CISiMe₃ en présence de LDA ou de Li-HMDS dans un mélange de THF et de solvants du type pentane, hexanes, cyclohexane, heptane, toluène. La réaction d'alkylation par couplage de l'organométallique de formule R₅MgHal ou R₅Li avec les dérivés silylés de formules (IVb) et/ou (IVa) s'effectue selon les conditions classiques connues de l'homme du métier.

L'invention a plus particulièrement pour objet un procédé tel que défini ci-dessus caractérisé en ce que le dérivé silylé est un dérivé de triméthylsilyle ce qui permet d'obtenir les énols silylés de formules (IVb) et/ou (IVa) dans lesquelles Ra, Rb et Rc sont identiques et représentent un méthyle.

L'invention a plus particulièrement pour objet un procédé tel que défini ci-dessus caractérisé en ce que =K représente une fonction céto protégée sous forme de cétal cyclique, tel que le 3,3-éthylènedioxy.

L'invention a plus particulièrement pour objet un procédé tel que défini ci-dessus caractérisé en ce que (ZO-) est en position para et Z représente un groupement R₄, avec n égal à 2.

L'invention a plus particulièrement pour objet un procédé tel que défini ci-dessus caractérisé en ce que R₁ et R₂ sont identiques et représentent un groupe alkyle linéaire, tels que les radicaux méthyle ou éthyle.

L'invention a tout particulièrement pour objet un procédé tel que défini ci-dessus caractérisé en ce que (ZO-) est en position méta ou para, et Z représente un radical alkyle linéaire, tel que le radical méthyle.

L'invention a également pour objet un procédé de préparation tel que défini plus haut de composés de formule (I) telle que définie plus haut dans laquelle A représente un groupement CH-X et Z représente un groupement R₄, comprenant les étapes suivantes ;
a) on soumet un composé de formule (II) : 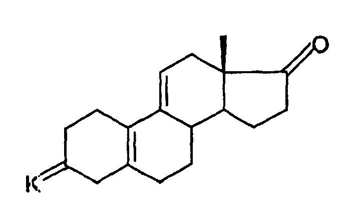 =K représentant une fonction céto protégée notamment sous forme de cétal, de thiocétal ou de cétal mixte,
   à l'action d'un agent réducteur du céto en 17 afin d'obtenir un composé de formule (VII) : 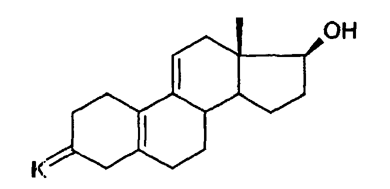
b) on traite le composé de formule (VII) avec un agent d'halogénation afin d'obtenir un composé de formule (VIII) : 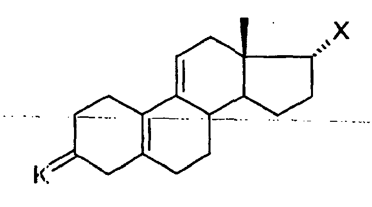 dans laquelle X représente un atome d'halogène,
c) on soumet le composé de formule (VIII) à l'action d'un réactif d'époxydation afin d'obtenir le mélange des composés de formules (III"a) et (III"b) : 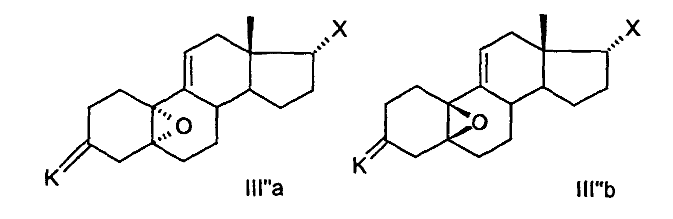
d) on soumet les composés de formules (III"a) et (III"b) à une réaction d'alkylation avec un dérivé organocuprate dérivé d'un organométallique de formule R₅MgHal ou R₅Li, Hal étant un atome d'halogène et R₅ représentant le groupement : 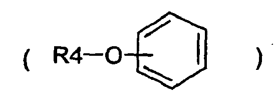 R₄ étant tel que défini plus haut, la liaison s'effectuant au niveau du phényle,
   puis à l'action d'un agent de déprotection afin d'obtenir les composés de formules (V"a), (V"b) et (V"c) : 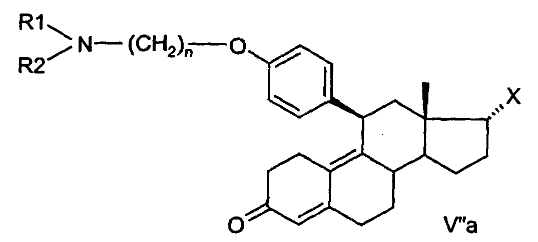 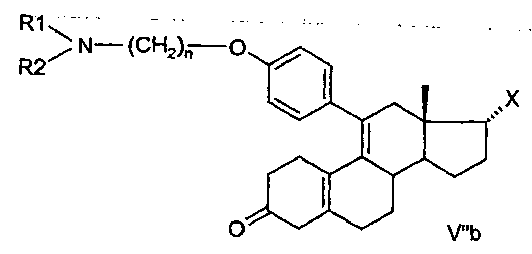 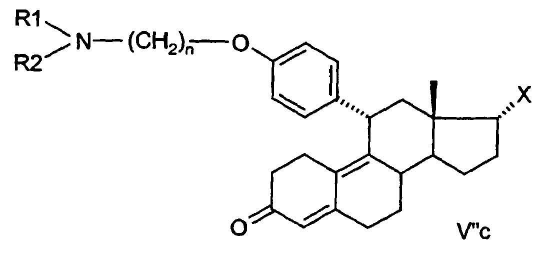
e) on traite les composés de formules (V"a), (V"b) et (V"c) avec un agent d'aromatisation pour obtenir le mélange des composés de formules (VI") et (I) dans laquelle A représente un groupement CH-X et Z représente un groupement R₄ , 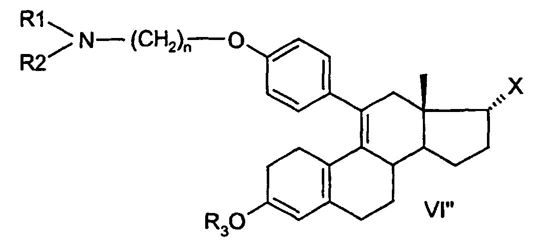 qui continuent de s'aromatiser pour obtenir le composé de formule (I) telle que définie plus haut.
f) le cas échéant on déprotège le produit obtenu au stade e pour obtenir un composé de formule (I) dans laquelle A représente un groupement CH-X, Z représente un groupement R₄, et R₃ représente un atome d'hydrogène, que l'on soumet le cas échéant à une salification et à une neutralisation.

La réduction du 17-céto en alcool s'effectue selon les méthodes classiques, notamment par action d'un borohydrure alcalin tel que le borohydrure de sodium dans le méthanol ou l'éthanol ou par action d'hydrure d'aluminium et de lithium dans le THF. Cette réaction permet d'obtenir notamment l'alcool en position 17 bêta. La réaction d'halogénation qui suit s'effectue notamment avec des réactifs tels que XSO₂C₄F₉ en présence d'une base encombrée telle que le DBU (diazabicycloundécène), X est de préférence le fluor. D'autres méthodes connues de l'homme du métier peuvent également être utilisées.

La réaction d'halogénation peut notamment s'effectuer en présence de fluorure de perfluorobutane sulfonyle, de complexe acide fluorhydrique/triéthylamine ((HF)₃, TEA) et de DBU.

La réaction d'alkylation avec un dérivé organocuprate dérivé d'un organométallique R₅MgHal ou R₅Li, Hal et R₅ étant tels que définis plus haut, s'effectue selon les méthodes classiques connues de l'homme du métier.

La réaction d'aromatisation suivie de la réaction de saponification s'effectue selon les méthodes classiques telles que décrites dans le brevet européen 0097572. Cette aromatisation peut s'effectuer de préférence en présence de bromure d'acétyle et d'anhydride acétique.

Le cas échéant, la déprotection du groupement acétyle formé s'effectue en général en présence d'une base forte, telle que la soude ou la potasse, dans un alcool, tel que le méthanol ou l'éthanol.

Les réactions de salification et de neutralisation sont effectuées par les méthodes classiques connues de l'homme du métier.

L'invention a plus particulièrement pour objet un procédé tel que défini ci-dessus caractérisé en ce que =K représente une fonction céto protégée sous forme de cétal cyclique, tel que le 3,3-éthylènedioxy.

L'invention a plus particulièrement pour objet un procédé tel que défini ci-dessus caractérisé en ce que X représente un atome de fluor.

L'invention a tout particulièrement pour objet un procédé tel que défini plus haut caractérisé en ce que R₁ et R₂ forment ensemble avec l'atome d'azote auquel ils sont liés un groupement 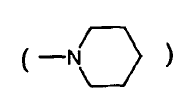 et n est égal à 2.

Les composés de formule (II) sont des composés connus ou aisément accessible à l'homme du métier. Notamment les composés de formule (II) dans laquelle =K est un groupement 3,3-éthylènedioxy, sont décrits dans l'article Crocq, V. et al.; Org. Process. Res. Dev. 1997, 1, 2.

Les composés de formules (IIIa) et (IIIb) sont des composés connus. Notamment les composés de formules (IIIa) et (IIIb) dans lesquelles =K est un groupement 3,3-éthylènedioxy et A représente une fonction céto, sont décrits dans l'article Larkin, J.P. et al.; Org. Process. Res. Dev. 2002, 6, 20.

L'invention a également pour objet à titre de produits intermédiaires nouveaux,
- le composé de formule générale (IV'b) telle que définie Plus haut et dans laquelle Ra, Rb et Rc sont identiques et représentent un méthyle,
- le composé de formule générale (VI') telle que définie plus haut et dans laquelle R₃ représente un groupement acyle.

L'invention a plus particulièrement pour objet à titre de produits intermédiaires nouveaux,
- le composé de formule générale (IV'b) telle que définie plus haut dans laquelle (ZO-) est en position para,
- le composé de formule générale (VI') telle que définie plus haut dans laquelle (ZO-) est en position para.

L'invention a également pour objet à titre de composés intermédiaires nouveaux,
- le composé de formule générale (VIII) dans laquelle =K représente le 3, 3-éthylènedioxy, et X représente un atome de fluor,
- les composés de formules générales (III"a) et (III"b) dans lesquelles =K représente le 3, 3-éthylènedioxy, et X représente un atome de fluor,
- les composés de formules générales (V"a), (V"b) et (V"c) dans lesquelles X représente un atome de fluor, n est égal à 2, et R₁ et R₂ forment ensemble avec l'atome d'azote auquel ils sont liés un groupement 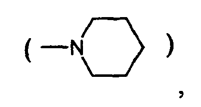
- le composé de formule générale (VI") dans laquelle R₃ représente un groupement acyle, X représente un atome de fluor, n est égal à 2, et R₁ et R₂ forment ensemble avec l'atome d'azote auquel ils sont liés un groupement 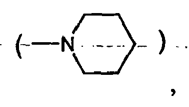
- le composé de formule générale (I) dans laquelle R₃ représente un groupement acyle, X représente un atome de fluor, n est égal à 2, et R₁ et R₂ forment ensemble avec l'atome d'azote auquel ils sont liés un groupement 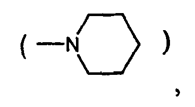
telles que définies plus haut.

### Partie expérimentale

### Exemple 1 : chlorhydrate de 11β-(4-(2-(diéthylamino) éthoxy)phényl)-estra-1,3,5(10)-trien-3-ol-17-one.

### Préparation 1 : bromure de 4-(2-(diéthylamino)éthoxy) phényle magnésium.

Sur du magnésium (tournures ; PM = 24.3 ; 7.5 g ; 1.13 eq.) vers 20°C sous agitation, on ajoute 25 ml d'une solution de bromure de 4-(2-(diéthylamino) éthoxy) benzène (74 g ; PM = 272.2) dans le THF (250 ml). Le mélange est agité vers 60°C jusqu'à la formation du réactif de Grignard (exothermique ; couleur grise). Le reste de la solution est ensuite ajouté avec précaution pendant environ 60 mn vers 58°C, et la suspension est agitée pendant 60 mn à la même température, puis on laisse refroidir. Le magnésien a une concentration environ 1.0 M.

### Stade a : 3,3-éthylènedioxy-5(10)-époxy-estr-9(11)-ène-17-one (mélange environ 2/1 d'isomères 5(10)-alpha et 5(10)-bêta).

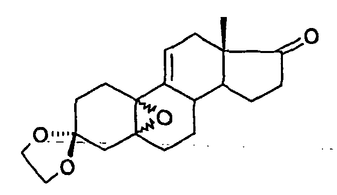

Le 3,3-éthylènedioxy-estra-5(10), 9(11)-diène-17-one (50 g ; M : 314,4 ; 0,159 mole), l'hexachloroacétone (98 % ; 2,5 ml ; 0.1 eq.), la pyridine (0,25 ml), le peroxyde d'hydrogène à 50 % (env. 18 M ; 15 ml ; 1.7 eq) et du dichlorométhane (250 ml) sont agités vigoureusement pendant 18 h à 20-25°C. Après réduction en présence de thiosulfate de sodium aqueux, lavages (eau) et extractions (dichlorométhane), la phase organique est concentrée jusqu'à un volume total d'environ 150 ml. Puis le dichlorométhane est remplacé par de l'éther isopropylique par une distillation en continu à volume constant, jusqu'à ce que la température intérieure atteigne 68°C. Le mélange est refroidi vers 20°C et on observe une précipitation spontanée du mélange d'époxydes. La suspension est refroidie puis agitée pendant 1 h à 0°C, et le produit est filtré, et séché sous vide pendant 18 h vers 40°C (44,6 g solide blanc ; rendement : 84,9 % ; pureté HPLC : 97 % ; mélange alpha/bêta environ 67/33) : C₂₀H₂₆O₄ ; MW : 330.4 . L'époxyde alpha peut être obtenu pur (solide blanc) par cristallisation dans l'acétonitrile ou l'acétate d'éthyle **:** Tf : 154°C ; [α]_{D} : + 133 ± 2.5° (c = 1% dans le chloroforme) ;

L'époxyde bêta peut être obtenu pur (solide blanc) par chromatographie (système éluant : heptane 50, acétate d'éthyle 50, pyridine 0.1) : Tf : 143°C , et il peut être recristallisé dans un mélange d'acétate d'éthyle et d'éther isopropylique : Tf : 162-163°C ; [α]_{D} : + 101.5 ± 2° (c = 1% dans le chloroforme) ; IR (CHCl₃, cm⁻¹) : 1735, 1640 ; RMN ¹H (CDCl₃, ppm) : 0,86 (s, 3H) ; 3,92 (m, 4H) ; 5.86 (m, 1H).

### Stade b : Ether d'énol silylé : 3,3 éthylenedioxy-5,10-époxy-17-triméthylsilyloxy-estra-9(11), 16(17)-diène (mélange environ 2/1 d'isomères 5,10-alpha et 5,10-bêta).

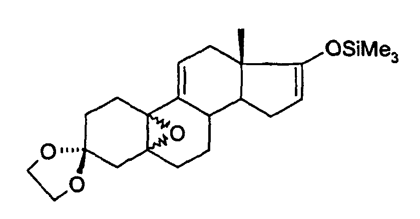

Du n-butyllithium (solution à 17% dans le cyclohexane ; 68 g ; 1.2 eq.) a été ajouté en 30 mn à une solution sous agitation de diisopropylamine (M : 101.2 ; d : 0.714 ; 30 ml ; 1.4 eq.) dans le THF anhydre(100 ml), à -10°C. La solution d'amidure lithié de diisopropylamine ainsi obtenue a été ajoutée pendant 30 mn vers 0°C à une solution d'époxyde préparé au stade A (50 g ; 0,151 mole) dans le THF (150 ml), et le mélange a été agité pendant 5 mn vers 0°C. Du triméthylchlorosilane (M : 108.6 ; d : 0.856 ; 27 ml ; 1.4 eq.) a été ajouté pendant 15 mn à 0°C, et le mélange a été agité pendant 1 h vers 0°C. Du méthanol (M : 32 ; d : 0.792 ; 5 ml ; 0.8 eq.) a été ajouté vers 0°C et le milieu a été agité pendant 30 mn vers 10°C puis coulé dans le mélange agité de dihydrate de dihydrogénophosphate de sodium (M :156 ; 26 g ; 1.1 eq.), d'eau et de toluène. Après décantation et lavage à l'eau, la phase toluénique a été séchée sur sulfate de sodium et concentrée sous vide jusqu'à un volume final de 100 ml environ. L'éther d'énol silylé a été engagé sous cette forme au stade suivant, mais un isolement par - extrait sec est possible C₂₃H₃₄O₄Si ; PM : 402.6. L'époxyde alpha (solide amorphe, Tf < 40°C) peut être obtenu pur par le même mode opératoire, mais partant d'époxyde alpha pur (stade A):
IR (CHCl₃, cm⁻¹) : 1621, 1254, 849
RMN ¹H (CDCl₃, ppm) : 0,19 (s, 9H) ; 0,80 (s, 3H) ; 3,85-4,00 (m, 4H) ; 4,47 (dd, J=1,5 et 1Hz, 1H) ; 6,03 (m, 1H) MS (m/z) : 402 (M⁺), 387 (M⁺-CH₃), 99

L'époxyde bêta (huile) peut être obtenu pur par le même mode opératoire, mais partant d'époxyde bêta pur (stade A) : RMN ¹H (CDCl₃, ppm) : 0,19 (s, 9H) ; 0,78 (s, 3H) ; 1,12 (m, 1H) ; 2,34 (d, J= 15 Hz, 1H) ; 3,90 (m, 4H) ; 4,48 (m, 1H) ; 5,84 (m, 1H).

### Stade c : mélange de 11-(4-(2-(diéthylamino)éthoxy) phényl)-estra-5(10),9(11)-diène-3,17-dione et de 11-alpha-(4-(2-(diéthylamino)éthoxy)phényl)-estra-4,9-diène-3,17-dione.

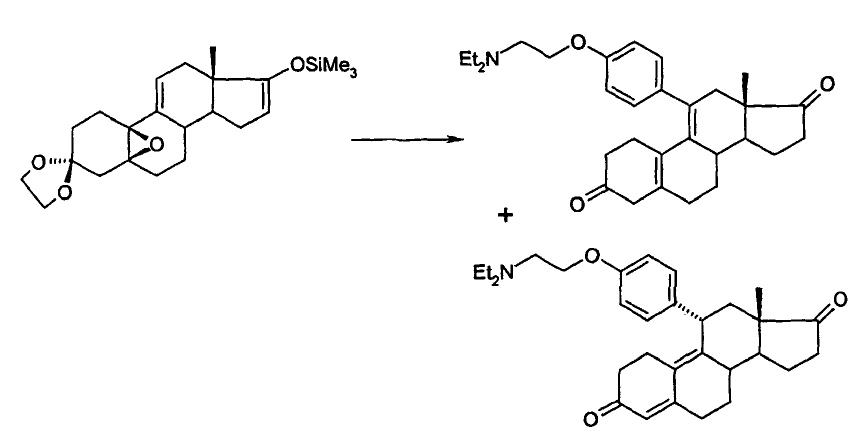

Le chlorure cuivreux (PM = 99.0 ; 509 mg ; 0.17 eq.) est ajouté vers 20°C à une solution d'éther d'énol silylé (isomère bêta pur) tel que préparé plus haut (stade B) (12.2 g ; 30.3 mmol) dans du THF (30 ml). On refroidit vers - 3°C et ajoute en maintenant la température la solution de magnésien telle que décrite plus haut (préparation 1) (61 ml ; environ 1 M ; 2 eq). Le milieu est agité pendant 3 h environ vers 20°C, puis versé dans un mélange de chlorure d'ammonium (50 g) dans de l'eau (200 ml). On extrait avec du dichlorométhane. La phase organique est ensuite lavée avec de l'eau et concentrée sous vide. Du dichlorométhane (80 ml) et l'eau (30 ml) sont ajoutés. Le mélange est refroidi à 0-5°C et de l'acide chlorhydrique à 36% (12 ml ; 4.75 eq.) est ajouté en 20 mn. Le milieu biphasique est agité vigoureusement pendant 2 h vers 0°C , puis dilué avec de l'eau (50 ml). La phase organique est décantée et lavée avec de l'eau. Les produits secondaires aminés sont éliminés dans la phase aqueuse acide alors que les sels d'acide chlorhydrique des énones restent dans le dichlorométhane. La phase organique est neutralisée par lavage avec une solution aqueuse d'hydrogénocarbonate de sodium (50 ml), lavée avec de l'eau, séchée sur sulfate de sodium puis concentrée sous vide. Le résidu (11 g) est chromatographié sur une colonne de silice (éluant : n-heptane 50, acétate d'éthyle 45, triéthylamine 5). On concentre les fractions à sec et obtient :
- 800 mg de 11-(4-(2-(diéthylamino) éthoxy)phényl)-estra-5(10),9(11)-diène-3,17-dione : C₃₀H₃₉NO₃ ; PM : 461.6 ;
   IR (CHCl₃, cm⁻¹) : 1733, 1712, 1607, 1568, 1508 ;
   RMN ¹H (CDCl₃, ppm) : 1,03 (s, 3H) ; 1,12 (t, J= 7 Hz, 6H) et 2,72 (q, J= 7 Hz, 4H) ; 2,94 (t, J= 6 Hz, 2H) ; 4,09 (t, J= 6 Hz, 2H) ; 6,82 et 7,07 (m, 4H) ; de 1,0 à 2,9 (m, 18H) ;
   MS (EI, m/z) : 461 (M⁺), 100.
- 444 mg de 11-alpha-(4-(2-(diéthylamino) éthoxy) phényl)-estra-4,9-diène-3,17-dione : C₃₀H₃₉NO₃ ; PM 461.6 :
   RMN ¹H (CDCl₃, ppm) : 1,02 (s, 3H) ; 1,12 (t, J= 7 Hz, 6H) et 2,69 (q, J= 7 Hz, 4H) ; 2,91 (t, J= 7 Hz, 2H) ; 4,05 (m, 3H) ; 5,72 (s, 1H) ; 6,79 et 6,95 (m, 4H) ; de 1,0 à 2,8 (m, 16H) ;
   MS (ESP, m/z) : 426 (MH⁺).

### Stade c : mélange de 11-bêta-(4-(2-(diéthylamino)éthoxy) phênyl)-estra-4,9-diène-3,17-dione, de 11-(4-(2-(diéthylamino)éthoxy)phényl)-estra-5(10),9(11)-diène-3,17-dione et de 11-alpha-(4-(2-(diéthylamino)éthoxy) phényl)-estra-4,9-diène-3,17-dione.

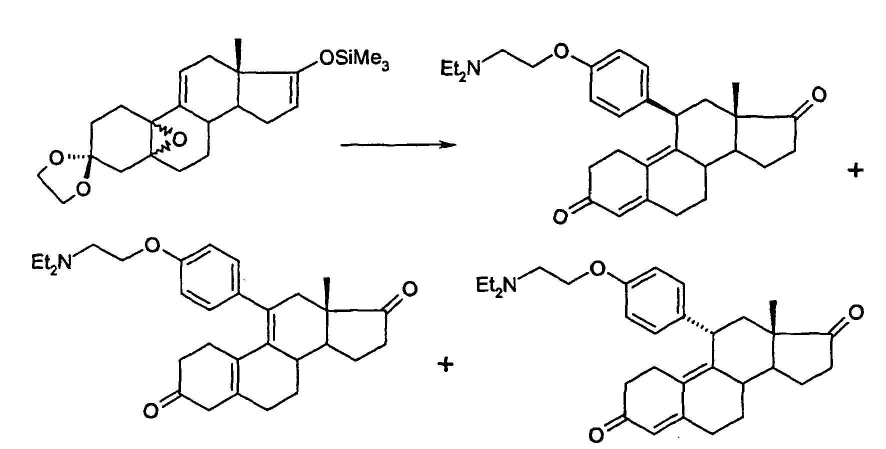

Le chlorure cuivreux (PM = 99.0 ; 2.24 g ; 0.25 eq.) est ajouté vers 20°C à une solution de magnésien telle que décrite plus haut (préparation 1) (182 ml ; environ 1M ; 2.0 eq). On agite 15 mn vers 20°C puis introduit une solution d'éther d'énol silylé (mélange bêta/ alpha environ 2/1) tel que préparé plus haut (stade B) (36.5g ; 90.7 mmol) dans du THF (120 ml). Le milieu est agité pendant 1 h environ vers 20°C, puis versé dans un mélange de chlorure d'ammonium (150 g) dans de l'eau (600 ml). On extrait avec du dichlorométhane. La phase organique est ensuite lavée avec de l'eau et concentrée sous vide. Du dichlorométhane (150 ml) et l'eau (75 ml) sont ajoutés, Le mélange est refroidi à 0-5°C et de l'acide chlorhydrique à 36% (45 ml ; 6 eq.) est ajouté en 45 mn, Le milieu biphasique est agité vigoureusement pendant 1.5 h vers 0 °C, puis dilué avec de l'eau (150 ml). La phase organique est décantée et lavée avec de l'eau. La phase organique est neutralisée par lavage avec une solution aqueuse saturée d'hydrogénocarbonate de sodium (150 ml), lavée avec de l'eau, séchée sur sulfate de sodium puis concentrée sous vide.

Le produit est une résine orange : 36 g ; rendement : 86 % : C₃₀H₃₉NO₃ ; PM : 461.6. La 11-bêta-(4-(2-(diéthylamino) éthoxy)phényl)-estra-4,9-diène-3,17-dione peut être isolée par cristallisation dans l'éther isopropylique (cristallisation d'autant plus efficace que la proportion alpha/ bêta dans le mélange initial d'époxydes était élevée). Les 11-(4-(2-(diéthylamino) éthoxy) phényl)-estra-5(10),9(11)-diène-3,17-dione et 11-alpha-(4-(2-(diéthylamino)éthoxy)phényl)-estra-4,9-diène-3,17-dione peuvent être isolées par chromatographie sur silice comme décrit dans l'exemple précédent.
11-bêta-(4-(2-(diéthylamino) éthoxy) phényl)-estra-4,9-diène-3,17-dione : Tf = 188°C ;
IR (CHCl₃, cm⁻¹) : 1735, 1658, 1609, 1581, 1509 ;
RMN ¹H (CDCl₃, ppm) : 0,56 (s, 3H) ; 1,06 (t, J= 7 Hz, 6H) et 2,63 (q, J= 7 Hz, 4H) ; 2,85 (t, J= 6 Hz, 2H) ; 4,01 (t, J= 6 Hz, 2H) ; 4,38 (dl, J= 7 Hz, 1H) ; 5,80 (s1, 1H) ; 6,82 et 7,07 (AA'BB', 4H) ; de 1,4 à 2,9 (m, 16H) ;
MS (EI, m/z) : 461 (M⁺) .

### Stade d : mélange de chlorhydrate de 3-acétyl-11-bêta-(4-(2-(diéthylamino)éthoxy)phényl)-estra-1,3,5(10)-trien-17-one et de chlorhydrate de 3-acétyl-11-(4-(2-(diéthylamino)éthoxy)phényl)-estra-3,5(10), 9(11)-trien-17-one.

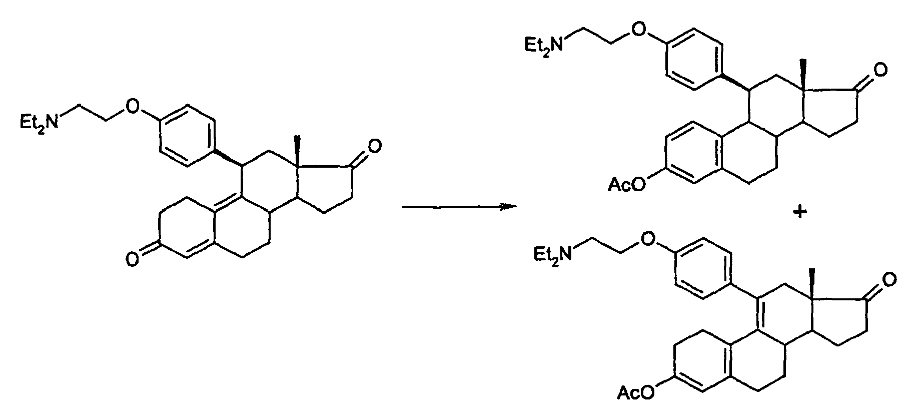

A une solution de 11-bêta-(4-(2-(diéthylamino) éthoxy) phényl)-estra-4,9-diène-3,17-dione préparée au stade c (10g ; 21,6 mmole) dans le dichlorométhane (40 ml) on ajoute de l'anhydride acétique (PM = 102.1 ; d = 1.09 ; 6.1 ml ; 3 eq.) et , en 5 mn, du bromure d'acétyle (PM = 123.0 ; d = 1.66 ; 6.1 ml; 3.8 eq.), à 20-25°C . La solution marron est agitée pendant 1 h à 20-25°C. On verse la solution en 30 mn environ dans une suspension d'hydrogenocarbonate de sodium (24.4 g) dans l'eau (100 ml) vers 20°C (dégagement de dioxyde de carbone). Le mélange est agité vigoureusement pendant 30 mn vers 20°C, puis la phase organique est décantée, lavée avec de l'eau séchée sur sulfate de sodium puis concentrée sous vide à sec. Le résidu (18 g) est chromatographié sur une colonne de silice (éluant : cyclohexane / acétate d'éthyle / triéthylamine 75 / 20 / 5). Chaque fraction est concentrée séparément à sec, reprise dans du dichlorométhane et acidifiée à pH = 1 par de l'acide chlorhydrique aqueux N. On décante la phase organique, sèche sur sulfate de sodium et concentre à sec. La fraction la moins polaire ainsi acidifiée donne le chlorhydrate de 3-acétyl-11-(4-(2 (diéthylamino) éthoxy) phényl)-estra-3,5(10),9(11)-trien-17-one (1.3 g ; solide jaune ; 10 %) : C₃₂H₄₂NO₄Cl; PM : 540.1.
IR (CHCl₃, cm⁻¹) : v 1736, 1664, 1606, 1570, 1507 ;
RMN ¹H (CDCl₃, ppm) : δ 1,02 (s, 3H) ; 1,47 (td, J= 7 Hz, 6H) et 3,27 (m, 4H) ; 2,10 (s, 3H) ; 3,47 (m, 2H) ; 4,51 (m, 2H) ; 5,52 (d, J= 1,5 Hz, 1H) ; 6,79 et 7,12 (AA' BB', 4H) ; 12,4 (sl, 1H mobile) ; de 1,0 à 2,6 (m, 16H) ;
SM (EI ; m/z) : 503 (M⁺) ; 461 ; 100 ; 86 ; 38 et 36 (HCl).

La fraction plus polaire acidifiée donne le chlorhydrate de 3-acétyl-11-bêta-(4-(2-(diéthylamino) éthoxy) phényle)-estra-1,3,5(10)-trien-17-one (6.09 g ; solide blanc ; 52 %) : C₃₂H₄₂NO₄Cl; PM : 540.1.
IR (CHCl₃, cm⁻¹) : v 1734, 1610, 1582, 1512, 1494 ;
RMN ¹H (CDCl₃, ppm) : δ 0,45 (s, 3H) ; 1,43 (t, J= 7 Hz, 6H) et 3,22 (m, 4H) ; 2,25 (s, 3H) ; 3,39 (m, 2H) ; 4,40 (m, 2H); 4,04 (d, J= 4,5 Hz, 1H) ; 6,63 et 6,99 (AA'BB', 4H) ; 6,65 (dd, J= 8,5 et 1,5 Hz, 1H) ; 6,86 (d, J= 1,5 Hz, 1H) ; 6,94 (d, J= 8,5 Hz, 1H) ; 12,3 (sl, 1H mobile) ; de 0,85 à 3,5 (m, 13H) ;
SM (EI ; m/z) : 503 (M⁺), 86, 38 et 36 (HCl).

### Stade e : 3-acétyl-11-bêta-(4-(2-(diéthylamino)éthoxy) phényl)-estra-3,5(10),9(11)-trien-17-one.

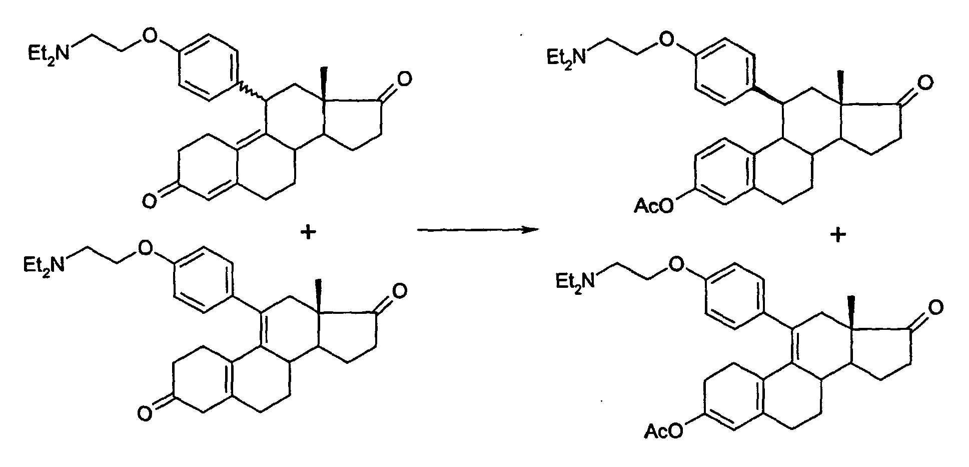

A la solution d'énones préparées au stade c (35 g ; 75.8 mmol) dans le dichlorométhane (250 ml) on ajoute de l'anhydride acétique (PM = 102.1 ; d = 1.09 ; 7.1 ml ; 1 eq.) et , en 20 mn, du bromure d'acétyle (PM = 123.0 ; d = 1.66 ; 14 ml; 2.5 eq.), à 20-25°C (addition exothermique). La solution marron est agitée pendant 5h à 20-25°C. On suit la disparition du 3-acétyl-triène par HPLC (Hypersil DBS 3 microns CN, 150 x 4.6 mm ; éluant : eau à 0.1% d'acide trifluoroacétique, acétone, méthanol 65/30/5 ; UV 210 nm). On verse la solution en 30 mn environ dans une suspension d'hydrogenocarbonate de sodium (93 g) dans l'eau (350 ml) vers 20°C (dégagement de dioxyde de carbone). Le mélange est agité vigoureusement une nuit vers 20°C, puis la phase organique est décantée, lavée avec de la soude 1 N (175 ml) et de l'eau puis concentrée jusqu'à un volume final de 100 ml. Le dichlorométhane est remplacé par du méthanol à volume constant par distillation sous vide progressif à environ 40°C. On conserve le produit en solution dans le méthanol.
C₃₂H₄₁NO₄ ; PM: 503.7.

### Stade f : chlorhydrate de 11-bêta-(4-(2-(diéthylamino) éthoxy)phényl)-estra-1,3,5(10)-trien-3-ol-17-one.

Sur la solution 3-acétyl-11-béta-(4-(2-(diéthylamino) éthoxy) phényl)-estra-3,5(10),9(11)-trien-17-one dans le méthanol obtenue au stade e, on ajoute en 10 mn environ vers 0°C une solution d'hydroxyde de potassium (PM = 56.0 ; 6.3 g ; 1.5 eq.) dans le méthanol (70 ml). Le milieu est agité pendant 2 h à 0-5°C, puis versé dans l'eau (175 ml) et le dichlorométhane (175 ml). La phase organique est décantée et lavée à l'eau. On ajoute de l'eau (175 ml) et de l'acide chlorhydrique à 36 % (16 ml ; 2.5 eq.) et agite pendant 5 mn environ en vérifiant le pH ( < 2). On décante la phase organique, la sèche sur sulfate de sodium, filtre et concentre jusqu'à obtention d'un volume final de 175 ml. On distille alors en maintenant le volume constant par introduction progressive de butan-2-one. Le produit attendu cristallise spontanément. La température en fin d'échange est de 78°C environ. On agite en laissant refroidir en 1 h environ, puis pendant 1 h vers 20°C. Le produit est filtré, lavé avec de la butan-2-one puis séché sous vide vers 70°C. (24.4 g solide beige ; Tf = 179°C ; rendement : 64.6 %) : C₃₀H₄₀ClNO₃ ; PM : 498.1 ;
IR (CHCl₃, cm⁻¹) : v 3601, 2456, 1733, 1610, 1584, 1511 ;
RMN ¹H (CDCl₃, ppm) : δ 0,42 (s, 3H), 1,31 (m, 6H), 3,16 (m, 4H), 3,31 (m, 2H), 3,96 (tl, 1H), 4,17 (m, 2H), 6,51 (m, 1H), 6,68 (m, 1H), 6,73 (m, 1H), 6,51 et 6,95 (AA'BB', 4H), 11,36 (sl; 1H) ;
SM (EI ; m/z) : 461 (M⁺), 446, 362, 86, 38 et 36 (HCl).

### Exemple 2 _{:} chlorhydrate de 11β-(4-(2-(diéthylamino) éthoxy)phényl)-estra-1,3,5(10)-trien-3-ol-17-one.

### Stade c : mélange de 11-bêta-(4-(2-(diéthylamino)éthoxy) phényl)-estra-4,9-diène-3,17-dione, de 11-(4-(2-(diéthylamino)éthoxy)phényl)-estra-5(10),9(11)-diène-3,17-dione et de 11-alpha-(4-(2-(diéthylamino)éthoxy) phényl)-estra-4,9-diène-3,17-dione.

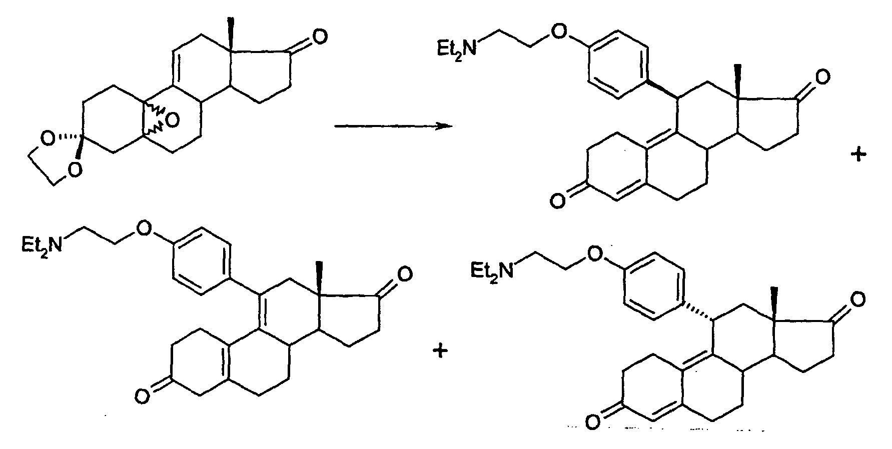

Sur du magnésium (tournures ; PM = 24.3 ; 11.8 g ; 3.2 eq.) vers 20°C sous agitation, on ajoute 25 ml d'une solution de bromure de 4-(2-(diéthylamino) éthoxy) benzène (123.6 g ; PM = 272.2 ; 3 eq.) dans le THF (250 ml). Le mélange est agité vers 60°C jusqu'à la formation du réactif de Grignard (exothermique ; couleur grise). Le reste de la solution est ensuite ajouté avec précaution pendant environ 60 mn vers 58°C, et la suspension est agitée pendant 60 mn à la même température, puis on laisse refroidir. Le chlorure cuivreux (PM = 99.0 ; 3.75 g ; 0.25 eq.) est ajouté vers 20°C et la suspension obtenue est agitée pendant 15 mn environ vers 20°C. Le mélange d'époxydes (stade A) (50 g ; 0.151 mole) solubilisé dans du THF (300 ml) est ajouté pendant 30 mn vers 20°C à la suspension. Le mélange est agité pendant 1 h vers 20°C, puis versé dans un mélange de chlorure d'ammonium (125 g) dans de l'eau (600 ml). On extrait avec du dichlorométhane. La phase organique est ensuite lavée avec de l'eau et concentrée sous vide. Du dichlorométhane (600 ml) et l'eau (125 ml) sont ajoutés. Le mélange est refroidi à 0-5°C et de l'acide chlorhydrique à 36% (100 ml ; 7.7 eq.) est ajouté en 20 mn. Le milieu biphasique est agité vigoureusement pendant 2 h vers 0°C, puis dilué avec de l'eau (250 ml). La phase organique est décantée et lavée avec de l'eau. Les produits secondaires aminés sont éliminés dans la phase aqueuse acide alors que les sels d'acide chlorhydrique des énones restent dans le dichlorométhane. La phase organique est neutralisée par lavage avec une solution aqueuse d'hydrogénocarbonate de sodium (25 g ; 2 eq.), lavée avec de l'eau, séchée sur sulfate de sodium puis concentrée sous vide. Le produit est sous forme semi-cristallisée : 65.3 g ; rendement : 93.5 % (à partir de l'époxyde 2) : C₃₀H₃₉NO₃ ; PM : 461.6.

### Stade e : 3-acétyl-11-bêta-(4-(2-(diéthylamino)éthoxy) phényl)-estra-3,5(10),9(11)-trien-17-one.

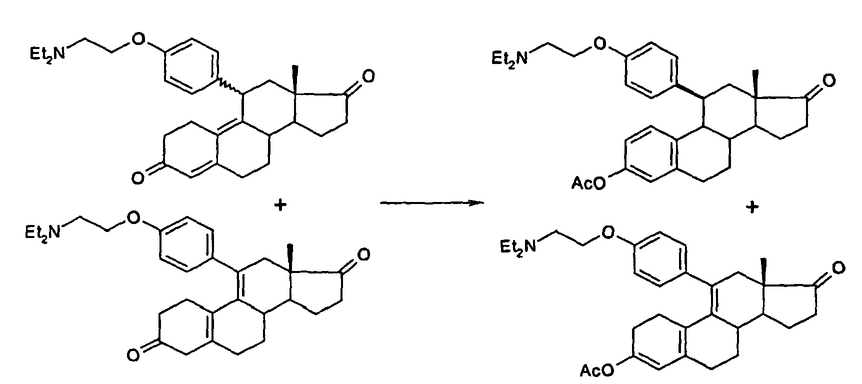

A une solution d'énones préparées au stade c (65.3 g ; 0.141 mole) dans le dichlorométhane (250 ml) on ajoute de l'anhydride acétique (PM = 102.1 ; d = 1.09 ; 14.2 ml ; 1.1 eq.) et , en 15 mn, du bromure d'acétyle (PM = 123.0 ; d = 1.66 ; 28 ml; 2.7 eq.), à 20-25°C (addition exothermique). La solution marron est agitée pendant 5 h à 20-25°C. On suit la disparition du 3-acétyl-triène par HPLC (Hypersil DBS 3 microns CN, 150 x 4.6 mm ; éluant : eau à 0.1% d'acide trifluoroacétique, acétone, méthanol 65/30/5 ; UV 210 nm). On verse la solution en 30 mn environ dans une suspension d'hydrogénocarbonate de sodium (127 g) dans l'eau (500 ml) vers 20°C (dégagement de dioxyde de carbone). Le mélange est agité vigoureusement une nuit vers 20°C, puis la phase organique est décantée, lavée avec de la soude 1 N (250 ml) et de l'eau (3 x 250 ml) puis concentrée jusqu'à un volume final de 150 ml. Le dichlorométhane est remplacé par du méthanol à volume constant par distillation sous vide progressif à environ 40°C. On conserve le produit en solution dans le méthanol. C₃₂H₄₁NO₄ ; PM : 503.7.

### Stade f : chlorhydrate de 11-bêta-(4-(2-(diéthylamino) éthoxy)phényl)-estra-1,3,5(10)-trien-3-ol-17-one.

Sur la solution de 3-acétyl-11-bêta-(4-(2-(diéthylamino) éthoxy) phényl)-estra-3,5(10),9(11)-trien-17-one dans le méthanol obtenue au stade e on ajoute en 10 mn environ vers 0°C une solution d'hydroxyde de potassium (PM = 56.0 ; 12.7 g ; 1.6 eq.) dans le méthanol (100 ml). Le milieu est agité pendant 1.5 h à 0-5°C, puis versé dans l'eau (250 ml) et le dichlorométhane (250 ml). La phase organique est lavée à l'eau. On acidifie par ajout d'eau et d'acide chlorhydrique à 36 % (26 ml ; 2.2 eq.) et on agite pendant 5 mn environ en vérifiant le pH (< 2). On décante la phase organique, la sèche sur sulfate de sodium, filtre et concentre jusqu'à l'obtention d'un volume final de 300 ml. On distille alors en maintenant le volume constant par introduction progressive de butan-2-one. Le produit attendu cristallise spontanément. La température en fin d'échange est de 78°C environ. On agite en laissant refroidir en 1 h environ, puis pendant 1 h vers 20°C Le produit est filtré, lavé avec de la butan-2-one puis séché sous vide vers 70°C (48 g solide beige ; Tf = 179°C ; rendement : 68.3 % pureté HPLC : 95 % ) :C₃₀H₄₀ClNO₃ ; PM 498.1. Mêmes données spectrales que celles citées dans l'exemple 1, stade f.

### Exemple 3 : 11-bêta-(4-(2-(diméthylamino)éthoxy) phényl)-estra-1,3,5(10)-trien-3-ol-17-one.

### Préparation 2 : bromure de 4-(2-(diéthylamino)éthoxy) phényle magnésium.

Sur du magnésium (tournures ; PM = 24.3 ; 2.2 g ; 1.2 eq.) en suspension dans 10 ml de THF anhydre on ajoute goutte à goutte une solution de bromure de 4-(2-(diméthylamino)éthoxy)benzène (18.3 g ; PM = 244.1) dans le THF (90 ml), sous agitation à 35-40 °C, après avoir amorcé la réaction par quelques gouttes de dibromoéthane. Après la fin de l'introduction, la solution grise obtenue est agitée pendant 90 mn à la même température, puis on laisse refroidir. Le magnésien a une concentration de 0.55 M (titre par iodométrie).

### Stade c : 3,3-éthylènedioxy-11-alpha-(4-(2-(diméthylamino)éthoxy)phényl)-estr-9-èn-5-bêta-ol-17-one.

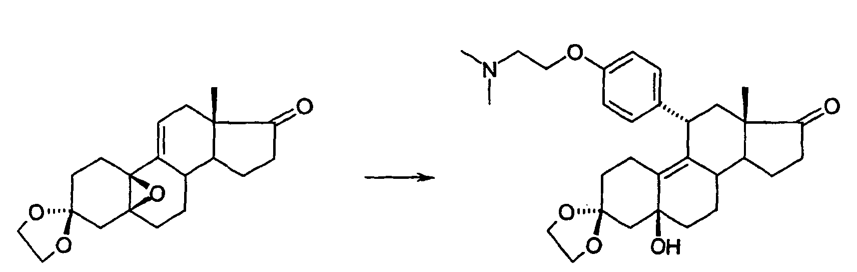

A une suspension de chlorure cuivreux anhydre (PM = 99 ; 0.5 g ; 0.33 eq.) dans du THF anhydre (20 ml) agitée durant 10 mn à 20 °C, on ajoute une solution de magnésien préparé ci-dessus (préparation 2) (82.5 ml ; 0.55 M ; 3 éq.). Le mélange est refroidi au bain de glace. On y ajoute sous agitation vers 0 °C et en 20 mn une solution de 3,3-éthylènedioxy-5-bêta,10-bêta -époxy-estr-9(11)-èn-17-one (4.95 g ; M : 330,42 ; 15 mmol) dans du THF anhydre (50 ml). Après 4 h d'agitation, le mélange est versé sur une solution aqueuse glacée et saturée de chlorure d'ammonium, le produit est extrait par de l'acétate d'éthyle. La phase organique est lavée par de l'eau salée, séchée sur sulfate de sodium puis concentrée sous vide. Le résidu (14 g) est chromatographié sur une colonne de silice (éluant : cyclohexane 40, acétate d'éthyle 40, triéthylamine 20). On concentre les fractions à sec pour obtenir :
- 5.7 g de 3,3-éthylènedioxy-11-alpha-(4-(2-(diméthylamino) éthoxy)phényl)-estr-9-èn-5-bêta-ol-17-one, rendement : 77 %, C₃₀H₄₁NO₅ ; PM : 495.6.

Par cristallisation (dissolution dans un petit volume de dichlorométhane puis addition d'éther isopropylique et légère concentration sous vide), essorage et séchage à 50 °C sous vide, on obtient :
- 4.24 g de cristaux blancs, Tf = 122 °C.
- IR (CHCl₃, cm⁻¹) ; 3510, 1735, 1609, 1578, 1510

### Stade d : 11-alpha-(4-(2-(diméthylamino)éthoxy)phényl)-estra-4,9-diène-3,17-dione et 11-(4-(2-(diméthylamino) éthoxy)phényl)-estra-5(10),9(11)-diène-3,17-dione.

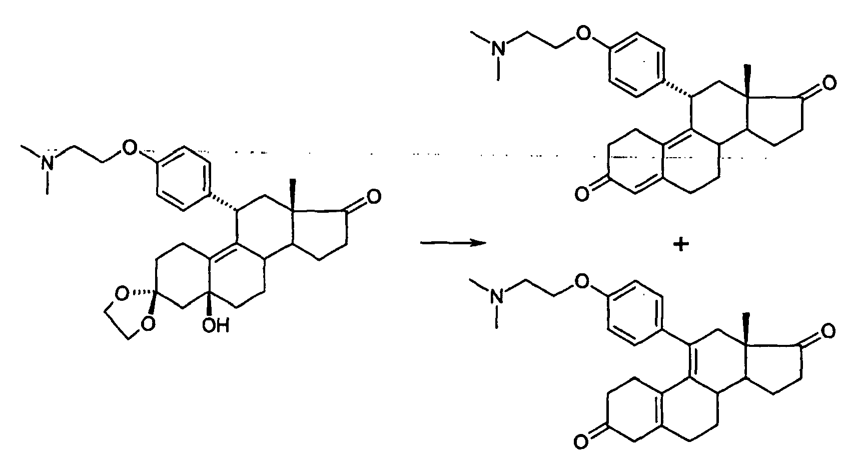

**Méthode a** : A une solution de 3,3-éthylènedioxy-11-alpha-(4-(2-(diméthylamino) éthoxy)phényl)-estr-9-èn-5-bêta-ol-17-one préparé au stade c (0.55 g ; 1.1 mmol) dans du méthanol (6 ml), on ajoute lentement en agitant de l'acide chlorhydrique 2 N (1 ml). Après 1 h 30 mn d'agitation, la solution est concentrée sous vide et le résidu est dissous dans de l'eau puis alcalinisé par une solution aqueuse saturée de bicarbonate de sodium. Le produit est extrait par de l'acétate d'éthyle. La phase organique est lavée par de l'eau salée, séchée sur sulfate de sodium puis concentrée sous vide. Le résidu (0.5 g) est chromatographié sur une colonne de silice (éluant : cyclohexane 40, acétate d'éthyle 40, triéthylamine 20). On concentre les fractions à sec pour obtenir :
- 0.325 g de 11-(4-(2-(diméthylamino)éthoxy)phényl)-estra-5(10),9(11)-diène-3,17-dione, rendement : 67 %, C₂₈H₃₅NO₃ ; PM : 433.6.
   IR (CHCl₃, cm⁻¹) : 1730, 1705, 1600 ;
- 0.025 g de 11-alpha-(4-(2-(diméthylamino) éthoxy) phényl)-estra-4,9-diène-3,17-dione, identique au produit obtenu par la méthode b, rendement : 5 %, C₂₈H₃₅NO₃ ; PM : 433.6.

**Méthode b :** A une solution de 3,3-éthylènedioxy-11-alpha-(4-(2-(diméthylamino)éthoxy)phényl)-estr-9-èn-5-bêta-ol-17-one préparé au stade c (0.5 g ; 1 mmol) dans de l'acide acétique (1 ml), on ajoute lentement en agitant de l'acide perchlorique à 55°Bé (2 ml). Après 1 h 45 mn de contact, le mélange est versé lentement en refroidissant dans une solution aqueuse saturée de bicarbonate de sodium. Le produit est extrait par de l'acétate d'éthyle. La phase organique est lavée par de l'eau salée, séchée sur sulfate de sodium puis concentrée sous vide. Le résidu (0.5 g) est chromatographié sur une colonne de silice (éluant : cyclohexane 40, acétate d'éthyle 40, triéthylamine 20). On concentre les fractions à sec pour obtenir :
- 0.16 g de 11-alpha-(4-(2-(diméthylamino)éthoxy) phényl)-estra-4,9-diène-3,17-dione, rendement : 36 %, C₂₈H₃₅NO₃ ; PM : 433.6.
   IR (CHCl₃, cm⁻¹) : 1739, 1652, 1609, 1580, 1510 ;
   RMN ¹H (CDCl₃, ppm) : 1,03 (s, 3H) ; 2.32 (s, 6H) ; 2.71 (t, J = 6 Hz, 2H) ; 4.03 (t, J = 6 Hz, 2H) ; 4.06 (t, J = 9 Hz, 1H) ; 5.72 (s, 1H) ; 6.83 et 6.99 (AA'BB', 4H).

### Stade e : 11-bêta-(4-(2-(diméthylamino)éthoxy)phényl)-estra-1,3,5(10)-trièn-3-ol-17-one.

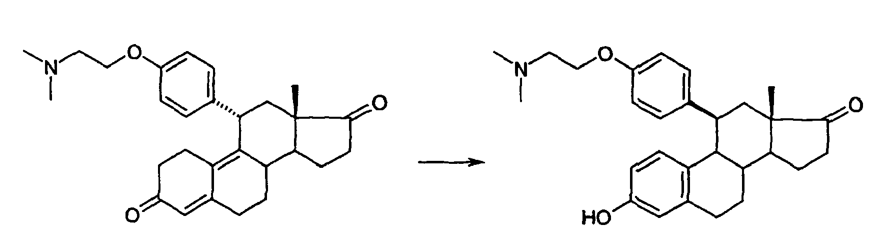

Le 11-alpha-(4-(2-(diméthylamino)éthoxy)phényl)-estra-4,9-diène-3,17-dione (0.2 g ; 0.46 mmol) préparé au stade d est dissous dans du chlorure de méthylène sec (2 ml). La solution est refroidie à 0°C puis on y ajoute goutte à goutte à cette température de l'anhydride acétique (0.2 ml ; 2.11 mmol ; 4.5 éq.) puis du bromure d'acétyle (0.1 ml ; 1.34 mmol ; 3 éq.). La solution obtenue est agitée à température ambiante durant 1 h 45 mn. On alcalinise avec précaution par une solution aqueuse saturée de bicarbonate de sodium. Le produit est extrait par du dichlorométhane. La phase organique est lavée par de l'eau salée, séchée sur sulfate de sodium puis concentrée sous vide. Le résidu (0.21 g) est dissous dans du méthanol (5 ml). On ajoute de la soude 2 N puis agite à température ambiante durant 45 mn. Le mélange est alors acidifié par de l'acide chlorhydrique 2 N, puis de nouveau alcalinisé par une solution aqueuse saturée de bicarbonate de sodium. Le produit est extrait par du dichlorométhane. La phase organique est lavée par de l'eau salée, séchée sur sulfate de sodium puis concentrée sous vide. Le résidu (0.19 g) est chromatographié sur une colonne de silice (éludant cyclohexane 40, acétate d'éthyle 40, triéthylamine 20). On concentre les fractions à sec pour obtenir :
- 0.13 g de 11-bêta-(4-(2-(diméthylamino)éthoxy) phényl)-estra-1,3,5(10)-trièn-3-ol-17-one, rendement : 65 %, C₂gH₃₅NO₃ ; PM : 433.6.
IR (CHCl₃, cm⁻¹) : 3595, 1735, 1610, 1580, 1512 ;
RMN ¹H (CDCl₃, ppm) : 0.47 (s, 3H) ; 2.33 (s, 6H) ; 3.94 (m, 2H) ; 3.99 (t, J = 4.5 Hz, 1H) ; 6.37 (dd, J = 2.5 et 8.5 Hz, 1H) ; 6.49 (d, J = 2.5 Hz, 1H) ; 6.77 (d, J = 8.5 Hz, 1H) ; 6.50 et 6.96 (AA'BB', 4H).

### Exemple 4 : 11-bêta-(4-(2-(1-pipêridinyl)éthoxy) phényl)-17-alpha-fluoro-estra-1,3,5(10)-trien-3-ol

### Stade a : Réduction 3,3-éthylènedioxy-estra-5(10), 9(11)-diéne-17-ol

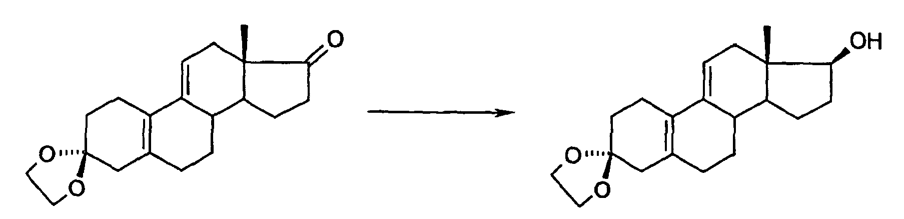

Sur une suspension de 3,3-éthylènedioxy-estra-5(10), 9(11)-diène-17-one (PM = 314.4 ; 100 g ; 318 mmol) dans le méthanol (1 litre) on introduit en 5 mn environ vers 2°C du borohydrure de sodium (PM = 37,8 ; 18.9 g ; 500 mmol) en solution dans de la soude 0.5 N (100 ml). On agite 2 h vers 2°C puis introduit en 15 mn environ vers 5°C de l'acétone (100 ml). On agite 1 h, et coule le milieu vers 20°C dans le mélange agité d'eau (2 litres), de chlorure de sodium (500 g) et d'acétate d'éthyle (400 ml). On décante et ré-extrait la phase aqueuse par de l'acétate d'éthyle. Les phases organiques réunies sont lavées à l'eau, séchées sur sulfate de sodium et concentrées à pression ordinaire à 500 ml. On poursuit la distillation en maintenant le volume constant par introduction progressive de 1,2- diméthoxyéthane (DME). La température en fin d'échange est 83°C. La solution est engagée telle quelle au stade suivant, mais un extrait sec conduit au produit attendu (résine). C₂₀H₂₈O₃ ; PM = 316.4) _{:}
IR (CHCl₃, cm⁻¹) : v 3613, 1638.
RMN ¹H (CDCl₃, ppm) : δ 0, 74 (s, 3H), 2,29 (sl, 2H), 3,78 (t, J= 8. 5 Hz, 1H), 3, 98 (m, 4H), 5, 57 (m, 1H), de 0.85 à 2-,-6 (m, 16H) ;

### Stade b : Fluoration

### 3,3-éthylènedioxy-17-alpha-fluoro-estra-5(10), 9(11)-diène

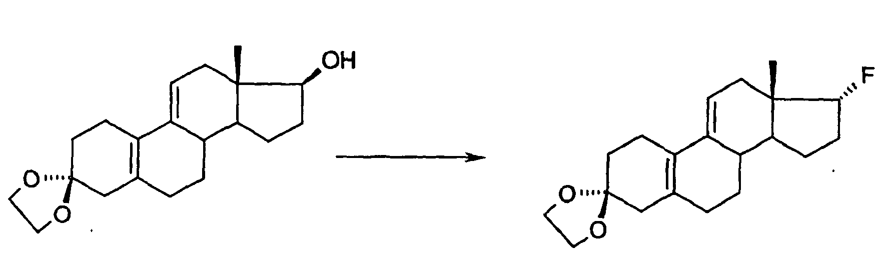

Sur la solution de 3,3-éthylènedioxy-estra-5(10), 9(11)-diêne-17-ol (PM = 316.4 ; 20 g ; 63.2 mmol) dans le DME (100 ml) obtenue au stade précédant, on introduit en 5 mn environ vers -10°C du fluorure de perfluorobutanesulfonyle (PM = 302,1 ; 41,4 g ; 137 mmol). On refroidit la suspension vers -40°C et introduit en 30 mn environ à cette température le complexe 3HF.TEA (PM= 161.2 ; 10.2 g ; 63.3 mmol). On agite 15 mn environ vers -40°C puis introduit en une heure environ à cette température la 1,8-diaza-bicyclo(5.4.0)-undéc-7-ène (DBU) (PM= 152 .2 ; 38, 4 g ; 252 mmol). On agite la suspension jaune 15 mn vers -40°C puis 3 h vers 2°C. On coule le milieu dans le mélange agité d'eau (400 ml), de chlorure d'ammonium (80 g) et d'acétate d'éthyle (140 ml), vers 10°C. On agite 30 mn, décante et ré-extrait avec de l'acétate d'éthyle. On lave les phases organiques réunies par de l'eau et de la soude 1N, sèche sur sulfate de sodium. On concentre sous vide puis introduit du dichlorométhane (300 ml). On introduit sur la solution de la silice (Merck Si 60 ; 60 g). On agite une heure vers 20°C, filtre la silice et rince au dichlorométhane (80 ml). On concentre le filtrat à pression ordinaire à 80 ml. On poursuit la distillation en maintenant le volume constant par introduction progressive d'isopropanol. La température en fin d'échange est 82°C. La solution est ramenée vers 20°C en amorce la cristallisation vers 63°C. La suspension est agitée une heure vers 20°C. On essore le produit blanc à 20°C et sèche sous vide une nuit vers 40°C . 12,34 g ; C₂₀H₂₇O₂F; PM = 318.4. Rdt= 61.3%. Tf = 100°C.
IR (CHCl₃, cm⁻¹) : v 1640, 1610 ;
RMN ¹H (CDCl₃, ppm) : δ 0,66 (d, J= 2.5 Hz, 3H), 3,99 (sl, 4H), 4, 59 (dd, J= 55 et 5 Hz; 1H), 5,60 (m, 1H), de 0, 8 à 2,6 (m, 18H);
SM (EI ; m/z) : 318 (M⁺), 298 (M⁺ - HF).

### Stade c : Epoxydation

### 3,3-éthylènedioxy-17-alpha-fluoro-5(10)-époxy-estr-9(11)-ène

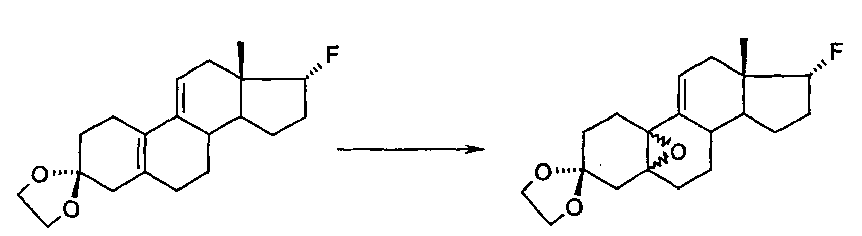

Le 3,3-éthylènedioxy-17-alpha-fluoro-estra-5(10), 9(11)-diène (100 g ; M : 318,4 ; 0,314 mole), l'hexafluoroacétone (trihydrate ; 8,75 ml ; 0,2 eq.), la pyridine (0,1 ml), le peroxyde d'hydrogène à 50 % (env. 18 M ; 30 ml ; 1.7 eq) et du dichlorométhane (1000 ml) sont agités vigoureusement pendant 18 h à 20-25°C. Après réduction en présence de thiosulfate de sodium aqueux, lavages (eau) et extractions (dichlorométhane), la phase organique est concentrée jusqu'à un volume total d'environ 400 ml. Puis le dichlorométhane est remplacé par du tétrahydrofurane par une distillation en continu à volume constant, jusqu'à ce que la température intérieure atteigne 66°C. La solution obtenue est refroidie et engagée telle quelle au stade suivant. (pureté HPLC _{:} 97% ; mélange alpha/bêta environ 70/30) : C₂₀H₂₇O₃F ; MW : 334.4. Après concentration à sec on peut cristalliser l'époxyde alpha dans l'heptane, et isoler l'époxyde bêta par chromatographie sur silice des liqueurs mères (éluant : cyclohexane 90- acétate d'éthyle 10).
Epoxyde alpha (Tf = 115°C):
IR (CHCl₃, cm⁻¹) : v 1642 ;
RMN ¹H (CDCl₃, ppm) : δ 0,66 (d, J= 2 Hz, 3H), 3, 85 à 3, 97 (m, 4H), 4,58 (dd, J= 55 et 5 Hz, 1H), 6,07 (dt, J= 5.5 et 2.5 Hz, 1H), 1 de 1,15 à 2,57 (m, 18H).
Epoxyde bêta (huile) :
IR (CHCl₃, cm⁻¹) : v 1642 ;
RMN ¹H (CDCl₃, ppm) : δ 0,64 (d, J= 1.5 Hz, 3H), 3,86 à 3 , 97 (m, 4H), 4, 59 (dd, J= 55 et 5 Hz; 1H), 5,88 (dt, J= 5.5 et 2 Hz, 1H), de 0,98 à 2,51 (m, 18H).

### Stade d : Alkylation

### Mélange de 17-alpha-fluoro-11-bêta-(4-(2-(1-pipéridinyl) éthoxy)phényl)-estra-4,9-dien-3-one et de 17-alpha-fluoro-11-(4-(2-(1-pipéridinyl)éthoxy)phényl)-estra-5(10), 9(11)-dien-3-one.

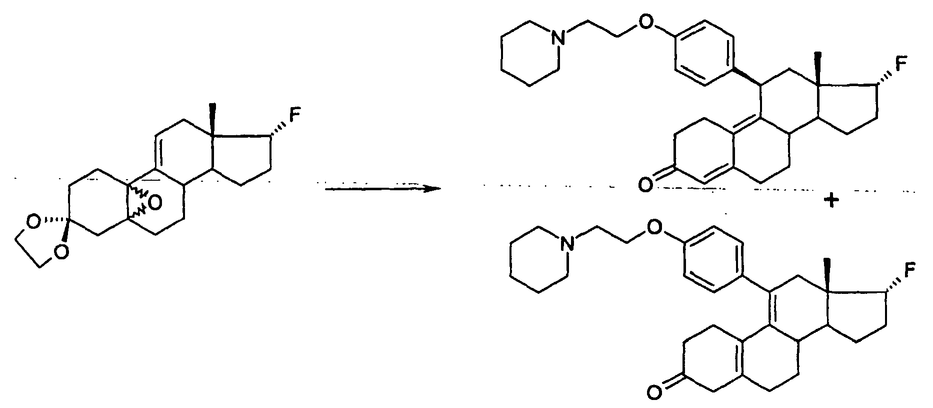

On introduit dans un réacteur 14,4 g de magnésium (tournures ; PM= 24.3 ; 593 mmol) puis 100 ml d'une solution 1-bromo-4-(2-(1-pipéridinyl)éthoxy)benzène (153, 3 g ; PM= 284,2 ; 539 mmol) dans le THF (560 ml). On chauffe vers 58°C et dès que le milieu devient gris, on introduit le reste de la solution vers 58°C en 1,5 h environ, puis maintient encore 2 h à cette température. On ramène la solution à 20°C et agite 18 h. On introduit le chlorure cuivreux (4, 43 g ; PM= 99.0 ; 44,7 mmol), agite 15 mn environ vers 20°C puis introduit en 30 mn vers 20°C la solution de 3,3-éthylènedioxy-17-alpha-fluoro-5(10)-époxy-estr-9(11)-ène (mélange alpha/bêta 7/3)(100 g ; PM= 334,4 ; 299 mmol) dans le THF décrite au stade précédant (stade c). On agite une heure vers 20°C et coule le milieu sur le mélange agité de chlorure d'ammonium (500 g), d'eau (2 litres) et de dichlorométhane (1 litre), vers 10°C. On décante et ré-extrait la phase aqueuse au dichlorométhane. On lave les phases organiques réunies à l'eau, et les concentre sous vide jusqu'à 200 ml environ. On refroidit la solution vers 2°C et introduit de l'eau (250 ml) puis de l'acide chlorhydrique concentré (36% ; 150 ml), toujours vers 2°C. On agite 1.5 h vers 2°C, puis dilue le milieu à l'eau (500 ml), décante et lave à l'eau jusqu'à pH > 4. On coule en 30 mn environ (formation de mousses) sur le mélange agité vers 20°C de bicarbonate de sodium (47 g ; PM= 84.0 ; 559 mol) dans l'eau (500 ml). On agite 30 mon et décante. On ré-extrait les phases aqueuses au dichlorométhane, lave à l'eau et sèche sur sulfate de sodium. La solution est décolorée par addition d'alumine (environ 200 g). On agite vers 20°C, filtre et rince au dichlorométhane. Le filtrat est concentré à pression ordinaire jusqu'à environ 700 ml, puis on continue la distillation en maintenant le volume constant par introduction progressive d'éther isopropylique. La température en fin d'échange est de 68°C. La cristallisation débute vers 63°C. On laisse refroidir vers 20°C et continue d'agiter encore 2 h vers 20°C. On essore le produit beige (61.9 g ; rendement : 43 %) : 17-alpha-fluoro-11-bêta-(4-(2-(1-pipéridinyl) éthoxy) phényl)-estra-4,9-dien-3-one. C₃₁H₄₀FNO₂ ; PM : 477. 7 ; Tf = 160°C ;
IR (CHCl₃, cm⁻¹) : 1656, 1608, 1508 ;
RMN ¹H (CDCl₃, ppm) : 0,35 (d, J= 2 Hz, 3H) ; 1,44 (m, 2H) ; 1,60 (m, 4H) ; 2, 50 (tl, J= 6 Hz, 4H) ; 2, 76 (t, 6 Hz, 2H) ; 4,07 (t, 6 Hz, 2H) ; 4,39 (m, 1H) ; 4,46 (dd, J= 55.5 et 5 Hz, 1H) ; 5,76 (sl, 1H) ; 6,82 et 7,07 (AA'BB', 4H) ; de 1, 2 à 4, 1 (m, 18H) ;
MS (EI ; m/z) : 477 (M⁺) ; 457 (M⁺-HF) ; 366 ; 346 ; 98.

Par chromatographie des liqueurs mères sur silice (éluant : heptane 55- acétate d'éthyle 40-triéthylamine 5), on obtient le 17-alpha-fluoro-11-(4-(2-(1-pipéridinyl)éthoxy)phényl)-estra-5(10), 9(11)-dien-3-one : C₃₁H₄₀FNO₂ ; PM : 477.7
IR (CHCl₃, cm⁻¹) : 1710, 1606, 1572, 1507 ;
RM13 ¹H (CDCl₃, ppm) _{:} 0, 81 (d, J= 2 Hz, 3H) ; 1, 46 (m, 2H) ; 1, 62 (m, 4H) ; 2,53 (m, 4H) ; 2, 80 (m, 2H) ; 4,10 (t, J= 6 Hz, 2H)-; 4,65-(dd, J= 55 et 5, 1H) ; 6,82 (m, 2H) ; 7,07 (m, 2H) ; de 1, 1 à 2, 85 (m, 18H) ;
MS (m/z) : 478 (MH⁺) ; 112.

### Stade e : Aromatisation

### 3-acétyl-11-bêta-(4-(2-(1-pipéridinyl)éthoxy)phényl)-17-alpha-fluoro-estra-1,3,5(10)-triène.

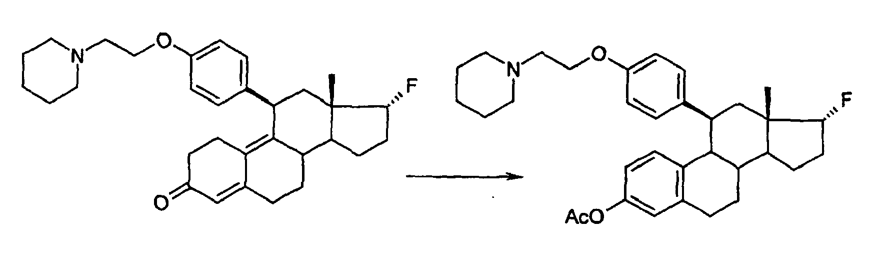

Sur une solution de 17-alpha-fluoro-11-bêta-(4-(2-(1-pipéridinyl) éthoxy) phényl)-estra-4,9-dien-3-one (38 g ; PM= 477,7 ; 79.5 mmol) (stade d) dans le dichlorométhane (152 ml) on ajoute de l'anhydride acétique (PM = 102.1 ; d = 1.09 ; 7.5 ml ; 1.0 eq.) et , en 15 mn, du bromure d'acétyle (PM = 123.0 ; d = 1.66 ; 14.7 ml; 2.5 eq.), à 20-25°C (addition exothermique). La solution marron est agitée pendant 5 h à 20-25°C. On verse la solution en 30 mn environ dans une suspension d'hydrogenocarbonate de sodium (45 g) dans l'eau (380 ml) vers 20°C (dégagement de dioxyde de carbone). Le mélange est agité vigoureusement une nuit vers 20°C, puis la phase organique est décantée, lavée avec de la soude 1 N (190 ml) et de l'eau, puis concentrée jusqu'à un volume final de 114 ml. Le dichlorométhane est remplacé par du méthanol à volume constant par distillation sous vide progressif à environ 40°C. On conserve le produit en solution dans le méthanol. C₃₃H₄₂FNO₃ ; PM : 519.8.

### Stade f : Saponification

### Chlorhydrate de 11-bêta-(4-(2-(1-pipéridinyl)éthoxy) phényl)-17-alpha-fluoro-estra-1,3,5(10)-trien-3-ol

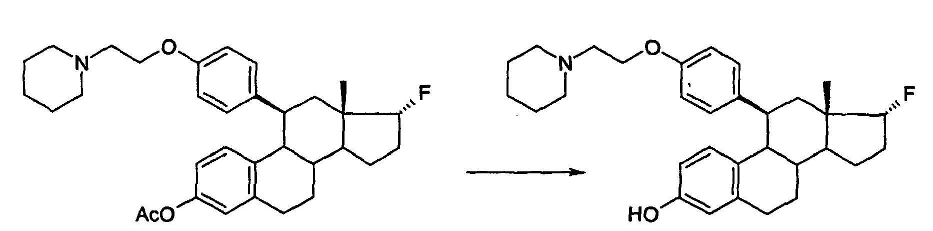

Sur la solution du dérivé fluoré dans le méthanol obtenue au stade e on ajoute en 10 mn environ vers 0°C une solution d'hydroxyde de potassium (PM = 56.0 ; 6.7 g ; 1.5 eq.) dans le méthanol (76 ml). Le milieu est agité pendant 45 mn à 0-5°C, puis versé dans l'eau (190 ml) et le dichlorométhane (190 ml). La phase organique est lavée à l'eau. On acidifie par ajout de méthanol (76 ml), d'eau (190 ml) et d'acide chlorhydrique à 36 % (17 ml ; 2.2 eq.) et agite pendant 5 mn environ en vérifiant le pH (< 2). On décante la phase organique, la sèche sur sulfate de sodium, filtre et concentre jusqu'à obtention d'un volume final de 190 ml. On distille alors à pression ordinaire en maintenant le volume constant par introduction progressive de dichlorométhane. Le produit attendu cristallise spontanément. On agite en laissant refroidir en 1 h environ, puis pendant 2 h vers 20°C. Le produit est filtré, lavé avec du dichlorométhane puis séché sous vide vers 40°C. (30.8 g solide beige ; rendement : 75.3 % pureté HPLC : 98 %) : C₃₁H₄₁ClFNO₂ ; PM : 514.1 ;
IR (CHCl₃, cm⁻¹) : v = 3599 ; 2467 ; 1609 ; 1583 ; 1511.
RMN ¹H (CDCl₃, ppm) : 0,22 (d, J= 1,5 Hz, 3H) ; 3,09 (m, 1H) ; 3,21 (m, 1H) ; 3,87 (m, 1H) ; 3,99 (m, 1H). 4,25 (m, 1H) ; 4,43 (dd, J= 56 et 5 Hz, 1H) ; 6,43 et 6,95 (AA'BB', 4H) ; 6,60 (dd, J= 8,5 et 1,5 Hz, 1H) ; 6.67 (d, J= 1,5 Hz, 1H) ; 6,78(d, J= 8,5 Hz, 1H) ; 11,4 (sl, 1H mobile) ; de 0,9 à 3,4 (m, 14H).
MS (ESP ; m/z) : 478 (MH⁺).

### Stade g : Neutralisation

### 11-bêta-(4-(2-(1-pipéridinyl)éthoxy)phényl)-17-alpha-fluoro-estra-1,3,5(10)-trien-3-ol

Sur une suspension de chlorhydrate de 11-bêta-(4-(2-(1-pipéridinyl) éthoxy) phényl)-17-alpha-fluoro-estra-1,3,5(10)-trien-3-ol (28 g ; PM= 514.1 ; 54.5 mmol) (stade f) dans le dichlorométhane (224 ml), on introduit vers 20°C du carbonate de sodium (PM= 106.0 ; 6.1 g) en solution aqueuse (112 ml). On agite 30 mn vers 20°C, décante et lave à l'eau. On sèche la phase organique sur sulfate de sodium, filtre et concentre le filtrat jusqu'à un volume résiduel de 140 ml. On ramène à 20°C et introduit de l'acétone (280 ml), puis de la silice (Merck Si 60 ; 42 g). On agite une heure vers 20°C, filtre et rince avec un mélange acétone-dichlorométhane 2/1.

On concentre le filtrat jusqu'à obtention d'un volume final de 224 ml. On distille alors à pression ordinaire en maintenant le volume constant par introduction progressive d'isopropanol. Le produit cristallise spontanément. On agite en laissant refroidir en 1 h environ, puis pendant 2 h vers 0°C. Le produit est filtré, lavé avec de l'isopropanol vers 0°C puis séché sous vide vers 40°C. (21.3 g solide blanc ; Tf = 180°C ; rendement : 82.1 % pureté HPLC : 99 %) : C₃₁H₄₀FNO₂ ; PM : 477.7 ;
IR⁻(CHCl₃, cm⁻¹) : v = 3598, 1610, 1581, 1512 ;
RMN ¹H(CDCl₃, ppm) : 0, 16 (d, J= 2,5 Hz, 3H) ; 1, 34 (m, 2H) ; 1,44 (m, 4H) ; 2,37 (m, 4H) ; 2,56 (t, J= 6 Hz, 2H) ; 3,91 (m, 2H) ; 3,95 (m, 1H) ; 4,44 (dd, J= 56 et 5 Hz, 1H) ; 6,31 (dd, J= 8,5 et 3 Hz, 1H) ; 6.46 (d, J= 3 Hz, 1H) ; 6,63 et 6, 97 (AA' BB' , 4H) ; 6, 71 (d, J= 8, 5 Hz, 1H) ; 8,95 (sl, 1H mobile) ; de 0,9 à 3,0 (m, 13H) .

## Revendications

1. Un procédé de préparation de composés de formule générale (I) : 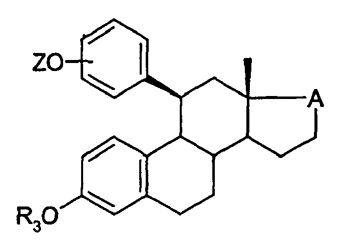 dans laquelle
Z représente un radical alkyle linéaire ou un groupement R₄. R₄ représente : 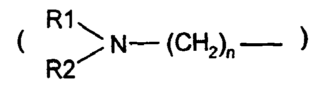 dans lequel n est un entier de 2 à 8, et
- soit R₁ et R₂, identiques ou différents représentent un groupement benzyle ou un radical alkyle, alkényle ou alkynyle linéaire, ramifié ou cyclique renfermant de 1 à 8 atomes de carbone
- soit R₁ et R₂ forment ensemble avec l'atome d'azote qui les porte un hétérocycle de 5 à 6 chaînons saturé ou insaturé, aromatique ou non aromatique renfermant éventuellement de 1 à 3 hétéroatomes additionnels et éventuellement accolé à un autre cycle,
A représente une fonction céto ou un groupement CH—X, X représentant un atome d'halogène,
R₃ représente un atome d'hydrogène ou un groupement protecteur de la fonction hydroxy,
comprenant les étapes suivantes ;
a) on soumet le mélange des composés de formules (IIIa) et (IIIb): 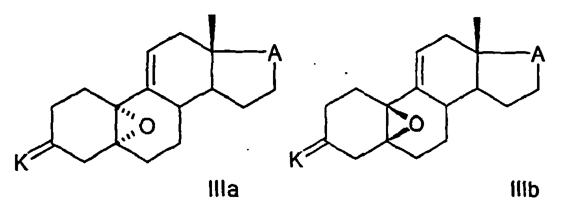 =K représentant une fonction céto protégée notamment sous forme de cétal, de thiocétal ou de cétal mixte,
à une réaction d'alkylation avec un dérivé organocuprate dérivé d'un organométallique de formule R₅MgHal ou R₅Li, Hal étant un atome d'halogène et généré de façon catalytique ou stoechiométrique, dans laquelle R₅ représente le groupement : 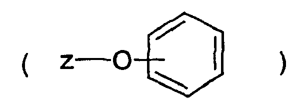 Z étant tel que défini plus haut, la liaison s'effectuant au niveau du phényle,
puis à l'action d'un agent de déprotection afin d'obtenir les composés de formules (Va), (Vb) et (Vc) : 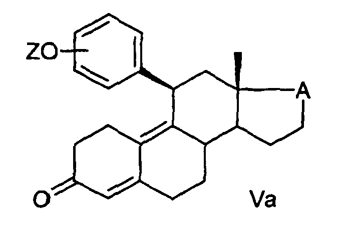 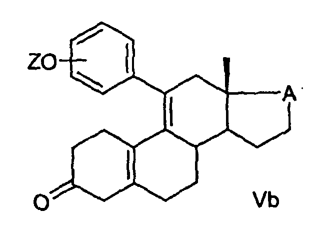 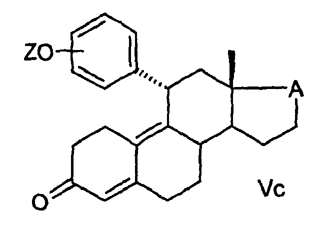
b) on traite les composés de formules (Va), (Vb) et (Vc) avec un agent d'aromatisation pour obtenir le mélange des composés de formules (VI) et (I) _{:} 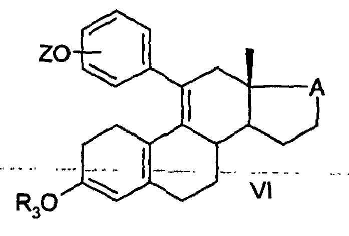 qui continuent à s'aromatiser pour obtenir le composé de formule (I), R₃ étant tel que défini plus haut.

2. Un procédé de préparation de composés de formule générale (I) : 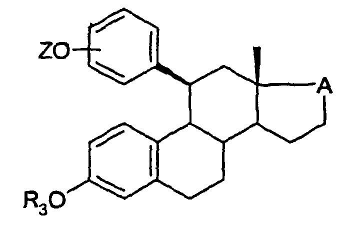 dans laquelle
Z représente un radical alkyle linéaire ou un groupement R₄. R₄ représente : 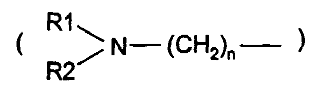 dans lequel n est un entier de 2 à 8, et
- soit R₁ et R₂, identiques ou différents représentent un groupement benzyle ou un radical alkyle, alkényle ou alkynyle linéaire, ramifié ou cyclique renfermant de 1 à 8 atomes de carbone
- soit R₁ et R₂ forment ensemble .avec l'atome d'azote qui les porte un hétérocycle de 5 à 6 chaînons saturé ou insaturé, aromatique ou non aromatique renfermant éventuellement de 1 à 3 hétéroatomes additionnels et éventuellement accolé à un autre cycle,
A représente une fonction céto ou un groupement CH—X, X représentant un atome d'halogène,
R₃ représente un atome d'hydrogène ou un groupement protecteur de la fonction hydroxy,
comprenant les étapes suivantes ;
a) on soumet le composé de formule (IIIb) : 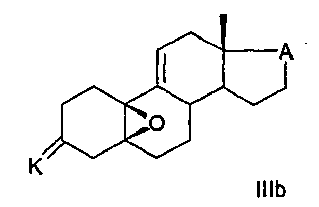 =K représentant une fonction céto protégée notamment sous forme de cétal, de thiocétal ou de cétal mixte,
à une réaction d'alkylation avec un dérivé organocuprate dérivé d'un organométallique de formule R₅MgHal ou R₅Li, Hal étant un atome d'halogène et généré de façon catalytique ou stoechiométrique, dans laquelle R₅ représente le groupement : 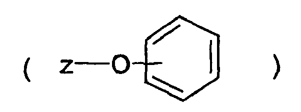 Z étant tel que défini plus haut, la liaison s'effectuant au niveau du phényle,
puis à l'action d'un agent de déprotection afin d'obtenir les composés de formules (Vb) et (Vc) : 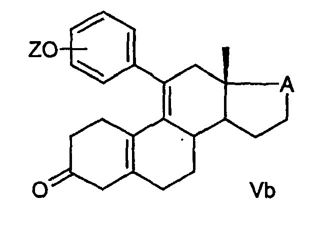 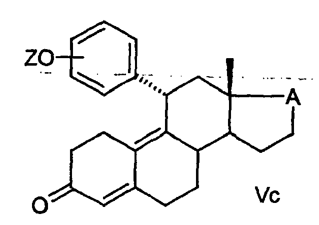
b) on traite les composés de formules (Vb) et (Vc) avec un agent d'aromatisation pour obtenir le mélange des composés de formules (VI) et (I) _{:} 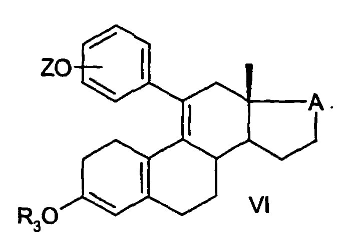 qui continuent à s'aromatiser pour obtenir le composé de formule (I), R₃ étant tel que défini plus haut.

3. Procédé de préparation selon la revendication 1, de composés de formule générale (I) dans laquelle A est une fonction céto, comprenant les étapes suivantes ;
a) on soumet un composé de formule (II) : 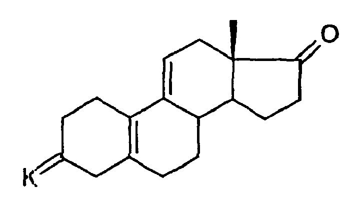 =K représentant une fonction céto protégée notamment sous forme de cétal, de thiocétal ou de cétal mixte,
à l'action d'un réactif d'époxydation afin d'obtenir le mélange des isomères alpha et bêta de formules (III'a) et (III'b) _{:} 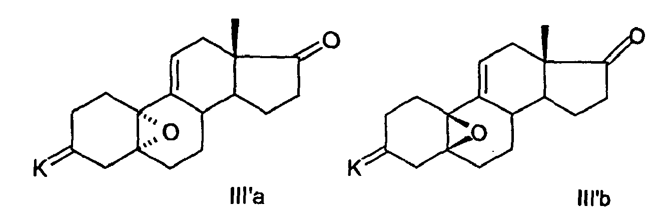
b) on soumet le mélange des composés de formules (III'a) et (III'b) à une réaction d'alkylation avec un dérivé organocuprate dérivé d'un organométallique de formule R₅MgHal ou R₅Li, Hal étant un atome d'halogène et R₅ étant tel que défini à la revendication 1, la liaison s'effectuant au niveau du phényle,
puis à l'action d'un agent de déprotection afin d'obtenir les composés de formules (V'a), (V'b) et (V'c): 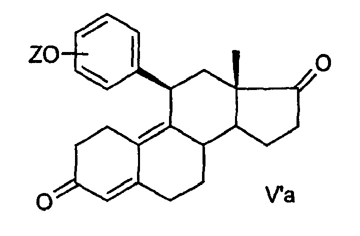 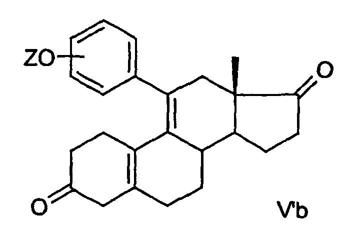 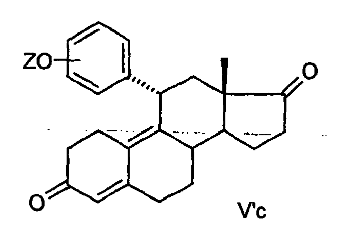
c) on traite les composés de formules (va), (V'b) et (V'c) avec un agent d'aromatisation pour obtenir le mélange des composés de formules (VI') et (I) dans laquelle A est une fonction céto : 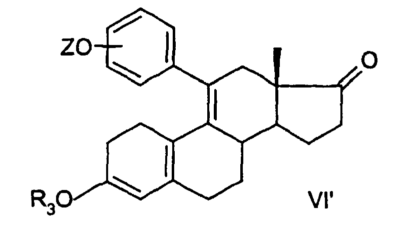 qui continuent à s'aromatiser pour obtenir le composé de formule (I) dans laquelle A est une fonction céto,
d) le cas échéant on déprotège le produit obtenu au stade c pour obtenir un composé de formule (I) dans laquelle A est une fonction céto et R₃ représente un atome d'hydrogène, que l'on soumet le cas échéant à une réaction de salification.

4. Procédé de préparation selon la revendication 2, de composés de formule générale (I) dans laquelle A est une fonction céto, comprenant les étapes suivantes ;
a) on soumet un composé de formule (II) : 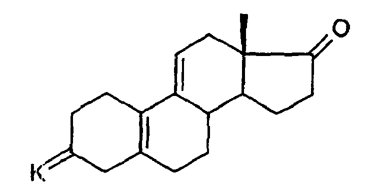 =K représentant une fonction céto protégée notamment sous forme de cétal, de thiocétal ou de cétal mixte,
à l'action d'un réactif d'époxydation afin d'obtenir l'isomère bêta de formule (III'b) : 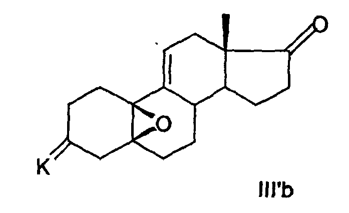
b) on soumet le composé de formule (III'b) à une réaction d'alkylation avec un dérivé organocuprate dérivé d'un organométallique de formule R₅MgHal ou R₅Li, Hal étant un atome d'halogène et R₅ étant tel que défini à la revendication 2, la liaison s'effectuant au niveau du phényle,
puis à l'action d'un agent de déprotection afin d'obtenir les composés de formules (V'b) et (V'c): 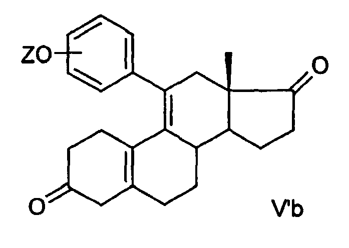 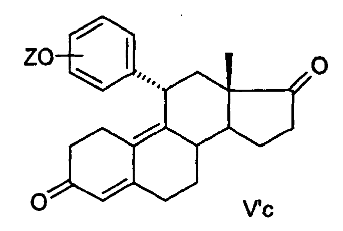
c) on traite les composés de formules (V'b) et (V'c) avec un agent d'aromatisation pour obtenir le mélange des composés de formules (VI') et (I) dans laquelle A est une fonction céto : 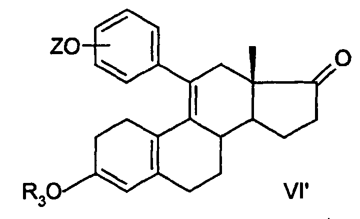
qui continuent à s'aromatiser pour obtenir le composé de formule (I) dans laquelle A est une fonction céto,
d) le cas échéant on déprotège le produit obtenu au stade c pour obtenir un composé de formule (I) dans laquelle A est une fonction céto et R₃ représente un atome d'hydrogène,
que l'on soumet le cas échéant à une réaction de salification.

5. Procédé selon la revendication 3 ou 4 **caractérisé en ce que** la réaction d'alkylation s'accompagne d'une réaction d'énolisation.

6. Procédé selon la revendication 3, **caractérisé en ce que** l'on traite les composés de formules (III'a) et (III'b)
avec un agent de silylation en présence d'une base, afin d'obtenir le mélange des énols silylés de formules (IVa) et (IVb) : 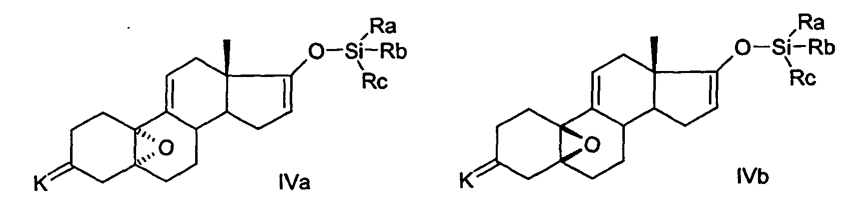 dans lesquelles Ra, Rb et Rc identiques ou différents représentent un radical alkyle renfermant de 1 à 4 atomes de carbone, ou un radical phényl,
que l'on fait réagir avec dérivé organocuprate tel que défini à la revendication 3, la liaison s'effectuant au niveau du phényle,
afin d'obtenir les composés de formules (IV'a) et (IV'b) qui sont isolés ou non isolés : 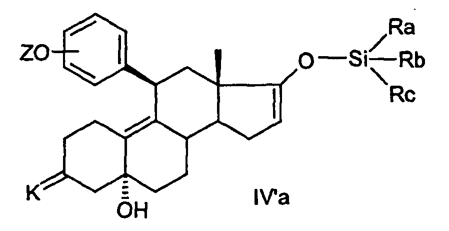 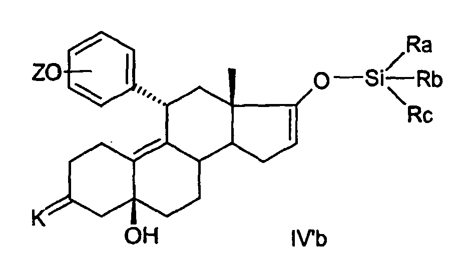 lesdits produits étant ensuite déprotégés pour obtenir les composés de formules (V'a), (V'b), (V'c) telles que définies à la revendication 3.

7. Procédé selon la revendication 4, **caractérisé en ce que** l'on traite le composé de formule (III'b) avec un agent de silylation en présence d'une base, afin d'obtenir l'énol silylé de formule (IVb) : 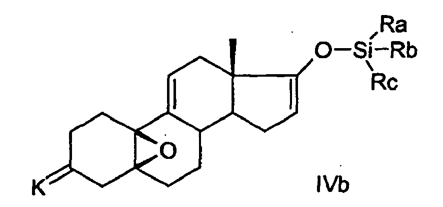 dans lesquelles Ra, Rb et Rc identiques ou différents représentent un radical alkyle renfermant de 1 à 4 atomes de carbone, ou un radical phényl,
que l'on fait réagir avec dérivé organocuprate tel que défini à la revendication 4, la liaison s'effectuant au niveau du phényle,
afin d'obtenir le composé de formule (IV'b) qui est isolé ou non isolé : 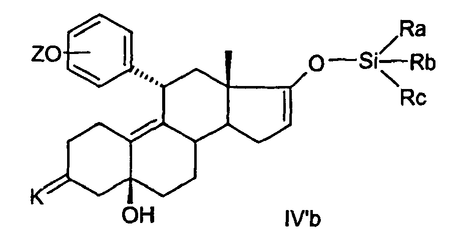 ledit produit étant ensuite déprotégé pour obtenir les composés de formules (V'b), (V'c) telles que définies à la revendication 4.

8. Procédé selon la revendication 6 ou 7 **caractérisé en ce que** le dérivé silylé est un dérivé de triméthylsilyle ce qui permet d'obtenir les énols silylés de formules (IVb) et/ou (IVa) dans lesquelles Ra, Rb et Rc sont identiques et représentent un méthyle.

9. Procédé selon la revendication 3, 4, 5 ou 8 **caractérisé en ce que** =K représente une fonction céto protégée sous forme de cétal cyclique, tel que le 3,3-éthylènedioxy.

10. Procédé selon la revendication 3, 4, 5, 8 ou 9 **caractérisé en ce que** (ZO-) est en position para et Z représente un groupement R₄, avec n égal 2.

11. Procédé selon la revendication 10, **caractérisé en ce que** R₁ et R₂ sont identiques et représentent un groupe alkyle linéaire, tels que les radicaux méthyle ou éthyle.

12. Procédé selon la revendication 3, 4, 5, 8, ou 9 **caractérisé en ce que** (ZO-) est en position méta ou para, et Z représente un radical alkyle linéaire, tel que le radical méthyle.

13. Procédé de préparation selon la revendication 1, de composés de formule (I) telle que définie à la revendication 1 dans laquelle A représente un groupement CH-X et Z représente un groupement R₄, comprenant les étapes suivantes ;
a) on soumet un composé de formule (II) : 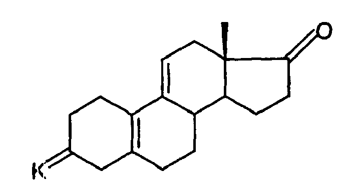 =K représentant une fonction céto protégée notamment sous forme de cétal, de thiocétal ou de cétal mixte,
à l'action d'un agent réducteur du céto en 17 afin d'obtenir un composé de formule (VII) : 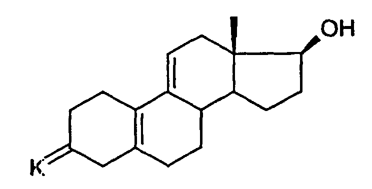
b) on traite le composé de formule (VII) avec un agent d'halogénation afin d'obtenir un composé de formule (VIII) _{:} 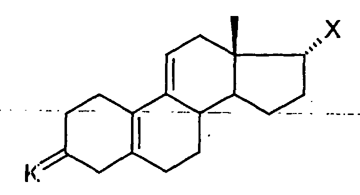 dans laquelle X représente un atome d'halogène,
c) on soumet le composé de formule (VIII) à l'action d'un réactif d'époxydation afin d'obtenir le mélange des composés de formules (III"a) et (III"b) . 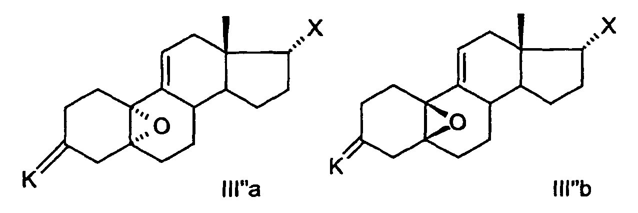
d) on soumet les composés de formules (III " a) et (III"b) à une réaction d'alkylation avec un dérivé organocuprate dérivé d'un organométallique de formule R₅MgHal ou R₅Li, Hal étant un atome d'halogène et R₅ représentant le groupement : 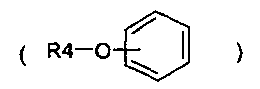 R₄ étant tel que défini à la revendication 1, la liaison s'effectuant au niveau du phényle,
puis à l'action d'un agent de déprotection afin d'obtenir les composés de formules (V" a), (V"b) et (V"c) : 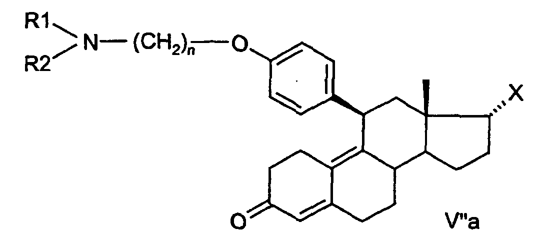 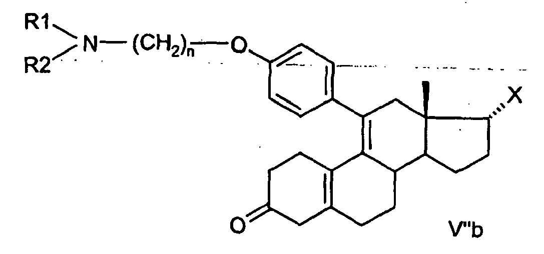 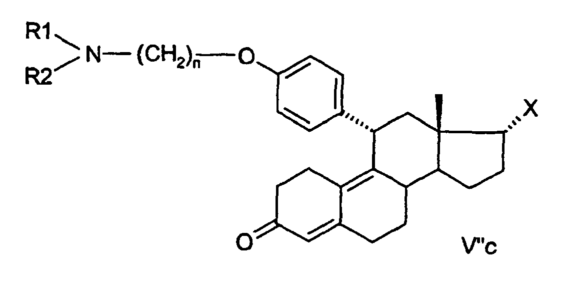
e) on traite les composés de formules (V"a), (V"b) et (V"c) avec un agent d'aromatisation pour obtenir le mélange des composés de formules (VI") et (I) dans laquelle A représente un groupement CH—X et Z représente un groupement R₄, 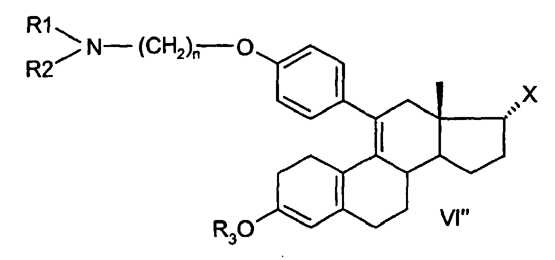 qui continuent de s'aromatiser pour obtenir le composé de formule (I) telle que définie plus haut.
f) le cas échéant on déprotège le produit obtenu au stade e pour obtenir un composé de formule (I) dans laquelle A représente un groupement CH—X, Z représente un groupement R₄, et R₃ représente un atome d'hydrogène, que l'on soumet le cas échéant à une salification et à une neutralisation.

14. Procédé selon la revendication 13 **caractérisé en ce que** =K représente une fonction céto protégée sous forme de cétal cyclique, tel que le 3,3-éthylènedioxy.

15. Procédé selon la revendication 13 ou 14 **caractérisé en ce que** X représente un atome de fluor.

16. Procédé selon la revendication 13, 14 ou 15 **caractérisé en ce que** R₁ et R₂ forment ensemble avec l'atome d'azote auquel ils sont liés un groupement 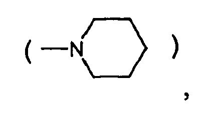 et n est égal à 2.

17. Procédé selon l'une quelconque des revendications 1 à 16 **caractérisé en ce que** l'agent d'aromatisation est le bromure d'acétyle en présence d'anhydride acétique.

18. Procédé selon l'une quelconque des revendications 1 à 12 **caractérisé en ce que** l'agent de déprotection utilisé pour obtenir les composés de formules (V'a), (V'c), ou (V'b) est un agent permettant une hydrolyse acide, tel que l'acide chlorhydrique ou l'acide perchlorique.

19. A titre de produits intermédiaires nouveaux,
- le composé de formule générale (IV'b) telle que définie à la revendication 6 et dans laquelle Ra, Rb et Rc sont identiques et représentent un méthyle,
- le composé de formule générale (VI') telle que définie à la revendication 3 et dans laquelle R₃ représente un groupement acyle.

20. Attitré de produits intermédiaires nouveaux,
- le composé de formule générale (IV'b) telle que définie à la revendication 19 dans laquelle (ZO-) est en position para,
- le composé de formule générale (VI') telle que définie à la revendication 19 dans laquelle (ZO-) est en position para.

21. A titre de composés intermédiaires nouveaux,
- le composé de formule générale (VIII) dans laquelle =K représente le 3, 3-éthylènedioxy, et X représente un atome de fluor,
- les composés de formules générales (III"a) et (III"b) dans lesquelles =K représente le 3, 3-éthylènedioxy, et X représente un atome de fluor,
- les composés de formules générales (V" a), (V" b) et (V" c) dans lesquelles X représente un atome de fluor, n est égal à 2, et R₁ et R₂ forment ensemble avec l'atome d'azote auquel ils sont liés un groupement 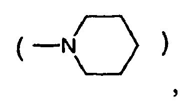
- le composé de formule générale (VI") dans laquelle R₃ représente un groupement acyle, X représente un atome de fluor, n est égal à 2, et R₁ et R₂ forment ensemble avec l'atome d'azote auquel ils sont liés un groupement 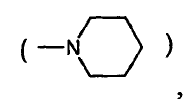
- le composé de formule générale (I) dans laquelle R₃ représente un groupement acyle, X représente un atome de fluor, n est égal à 2, et R₁ et R₂ forment ensemble avec l'atome d'azote auquel ils sont liés un groupements 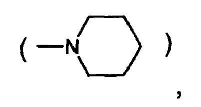 telles que définies à la revendication 13.

## Claims

1. Method for preparing compounds of general formula (I): 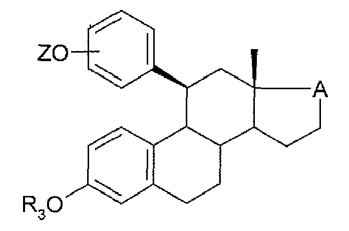 in which
Z represents a linear alkyl radical or a group R₄. R₄ represents : 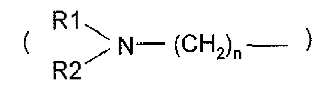 in which n is an integer from 2 to 8, and
- either R₁ and R₂, which are identical or different, represent a benzyl group or a linear, branched or cyclic alkyl, alkenyl or alkynyl radical containing from 1 to 8 carbon atoms,
- or R₁ and R₂ form together with the nitrogen atom carrying them a saturated or unsaturated, aromatic or nonaromatic 5- to 6-membered heterocycle optionally containing from 1 to 3 additional heteroatoms and optionally joined to another ring,
A represents a keto functional group or a group CH—X, X representing a halogen atom,
R₃ represents a hydrogen atom or a group protecting the hydroxyl functional group,
comprising the following steps :
a) the mixture of the compounds of formulae (IIIa) and (IIIb): 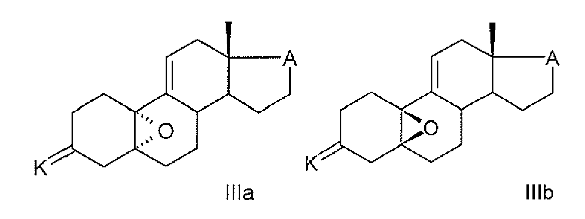 =K representing a keto functional group protected in particular in ketal, thioketal or mixed ketal form,
is subjected to an alkylation reaction with an organocuprate derivative derived from an organometallic compound of formula R₅MgHal or R₅Li, Hal being a halogen atom and generated catalytically or stoichiometrically, in which R₅ represents the group : 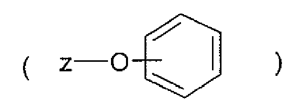 Z being as defined above, the bonding taking place on the phenyl,
and then to the action of a deprotecting agent so as to obtain the compounds of formulae (Va), (Vb) and (Vc) : 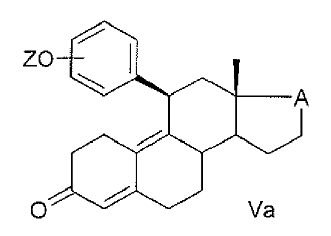 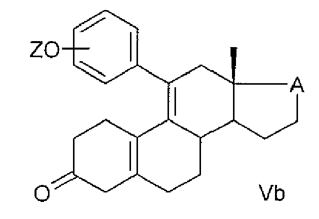 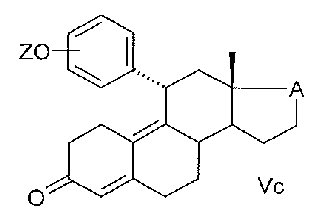
b) the compounds of formulae (Va), (Vb) and (Vc) are treated with an aromatisation agent so as to obtain the mixture of the compounds of formulae (VI) and (I) _{:} 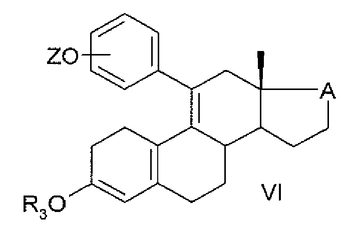 which continue to undergo aromatisation so as to obtain the compound of formula (I), R₃ being as defined above.

2. Method for preparing compounds of general formula (I) : 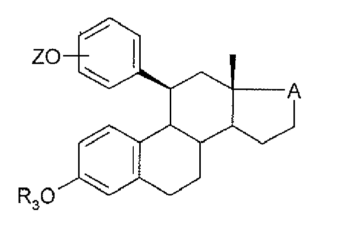 in which
Z represents a linear alkyl radical or a group R₄. R₄ represents: 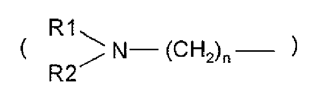 in which n is an integer from 2 to 8, and
- either R₁ and R₂, which are identical or different, represent a benzyl group or a linear, branched or cyclic alkyl, alkenyl or alkynyl radical containing from 1 to 8 carbon atoms,
- or R₁ and R₂ form together with the nitrogen atom carrying them a saturated or unsaturated, aromatic or nonaromatic 5- to 6-membered heterocycle optionally containing from 1 to 3 additional heteroatoms and optionally joined to another ring,
A represents a keto functional group or a group CH—X,
X representing a halogen atom,
R₃ represents a hydrogen atom or a group protecting the hydroxyl functional group,
comprising the following step
a) the compound of formula (IIIb): 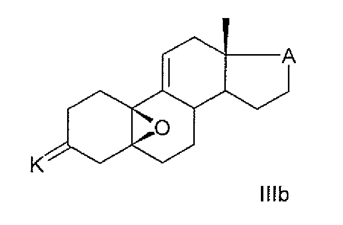 =K representing a keto functional group protected in particular in ketal, thioketal or mixed ketal form,
is subjected to an alkylation reaction with an organocuprate derivative derived from an organometallic compound of formula R₅MgHal or R₅Li, Hal being a halogen atom and generated catalytically or stoichiometrically, in which R₅ represents the group : 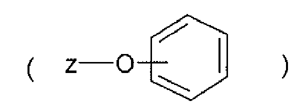 Z being as defined above, the bonding taking place on the phenyl,
and then to the action of a deprotecting agent so as to obtain the compounds of formulae (Vb) and (Vc) : 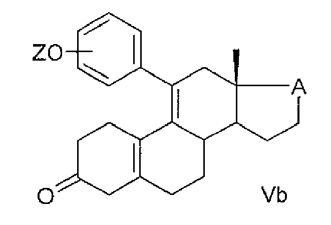 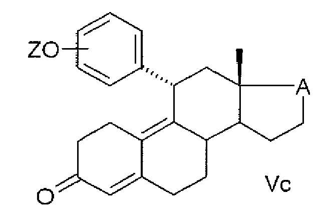
b) the compounds of formulae (Vb) and (Vc) are treated with an aromatisation agent so as to obtain the mixture of the compounds of formulae (VI) and (I) _{:} 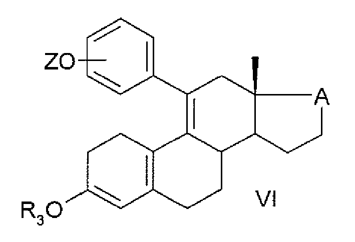 which continue to undergo aromatisation so as to obtain the compound of formula (I), R₃ being as defined above.

3. Method for preparing, according to claim 1, compounds of general formula (I) in which A is a keto functional group, comprising the following steps :
a) a compound of formula (II) _{:} 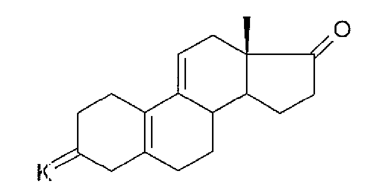 =K representing a keto functional group protected in particular in ketal, thioketal or mixed ketal form,
is subjected to the action of an epoxidation reagent so as to obtain the mixture of the alpha and beta isomers of formulae (III' a) and (III' b) . 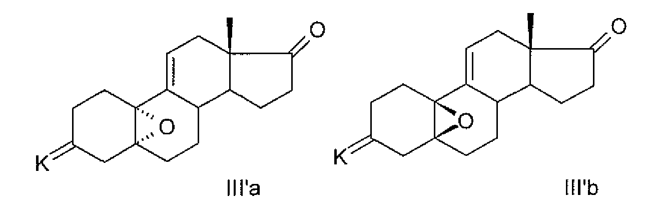
b) the mixture of the compounds of formulae (III'a) and (III'b) is subjected to an alkylation reaction with an organocuprate derivative derived from an organometallic compound of formula R₅MgHal or R₅Li, Hal being a halogen atom and R₅ being as defined in claim 1, the bonding taking place on the phenyl,
and then to the action of a deprotecting agent so as to obtain the compounds of formulae (V'a), (V'b) and (V'c) : 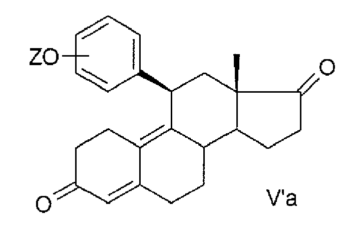 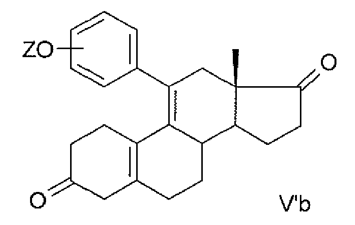 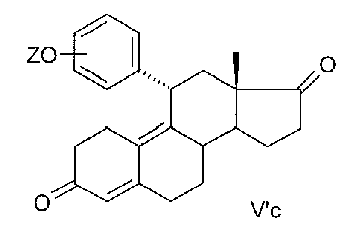
c) the compounds of formulae (V'a), (V'b) and (V'c) are treated with an aromatisation agent so as to obtain the mixture of the compounds of formulae (VI') and (I) in which A is a keto functional group : 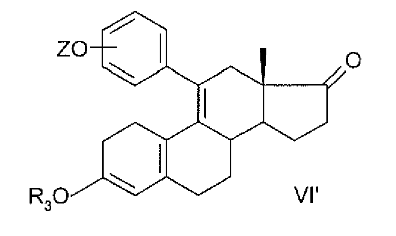 which continue to undergo aromatisation so as to obtain the compound of formula (I) in which A is a keto functional group,
d) where appropriate, the product obtained in step c is deprotected so as to obtain a compound of formula (I) in which A is a keto functional group and R₃ represents a hydrogen atom,
which is subjected, where appropriate, to a salification reaction.

4. Method for preparing, according to claim 2, compounds of general formula (I) in which A is a keto functional group, comprising the following steps :
a) a compound of formula (II) _{:} 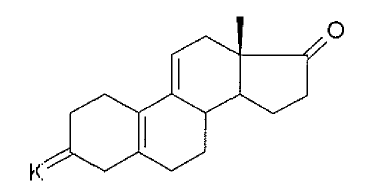 =K representing a keto functional group protected in particular in ketal, thioketal or mixed ketal form,
is subjected to the action of an epoxidation reagent so as to obtain the beta isomer of formula (III'b) _{:} 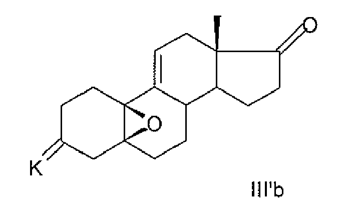
b) the compound of formula (III'b) is subjected to an alkylation reaction with an organocuprate derivative derived from an organometallic compound of formula R₅MgHal or R₅Li, Hal being a halogen atom and R₅ being as defined in claim 2, the bonding taking place on the phenyl,
and then to the action of a deprotecting agent so as to obtain the compounds of formulae (V'b) and (V'c): 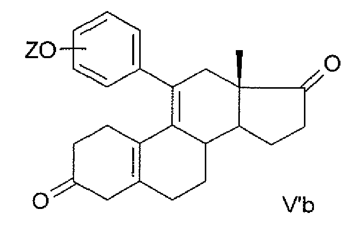 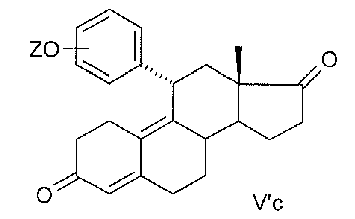
c) the compounds of formulae (V'b) and (V'c) are treated with an aromatisation agent so as to obtain the mixture of the compounds of formulae (VI') and (I) in which A is a keto functional group : 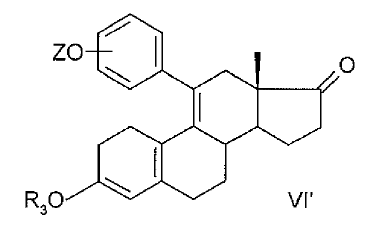 which continue to undergo aromatisation so as to obtain the compound of formula (I) in which A is a keto functional group,
d) where appropriate, the product obtained in step c is deprotected so as to obtain a compound of formula (I) in which A is a keto functional group and R₃ represents a hydrogen atom,
which is subjected, where appropriate, to a salification reaction.

5. Method according to claim 3 or 4, **characterised in that** the alkylation reaction is accompanied by an enolisation reaction.

6. Method according to claim 3, **characterised in that** the compounds of formulae (III'a) and (III'b) are treated with a silylating agent in the presence of a base, so as to obtain the mixture of silylated enols of formulae (IVa) and (IVb) : 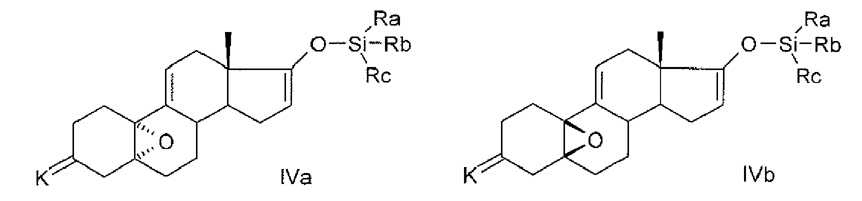 in which, Ra, Rb and Rc, which are identical or different, represent an alkyl radical containing from 1 to 4 carbon atoms, or a phenyl radical,
which is reacted with an organocuprate derivative as defined in claim 3, the bonding taking place on the phenyl,
so as to obtain the compounds of formulae (IV'a) and (IV'b) which are isolated or not isolated : 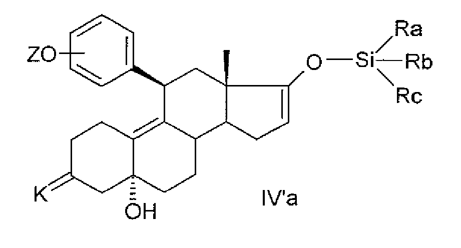 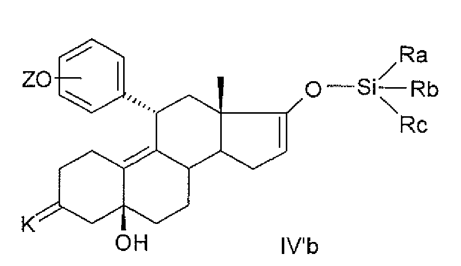 said products then being deprotected so as to obtain the compounds of formulae (V'a), (V'b), (V'c) as defined in claim 3.

7. Method according to claim 4, **characterised in that** the compound of formula (III'b)
is treated with a silylating agent in the presence of a base, so as to obtain the silylated enol of formula (IVb) : 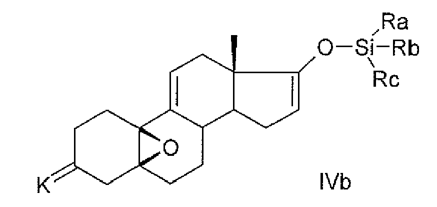 in which Ra, Rb and Rc, which are identical or different, represent an alkyl radical containing from 1 to 4 carbon atoms, or a phenyl radical,
which is reacted with an organocuprate derivative as defined in claim 4, the bonding taking place on the phenyl,
so as to obtain the compound of formula (IV'b) which is isolated or not isolated : 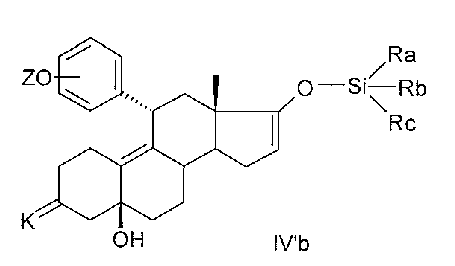 said product then being deprotected so as to obtain the compounds of formulae (V'b), (V'c) as defined in claim 4.

8. Method according to claim 6 or 7, **characterised in that** the silylated derivative is a trimethylsilyl derivative, which makes it possible to obtain the silylated enols of formulae (IVb) and/or (IVa) in which Ra, Rb and Rc are identical and represent a methyl.

9. Method according to claim 3, 4, 5 or 8, **characterised in that** =K represents a keto functional group protected in the form of a cyclic ketal, such as 3,3-ethylenedioxy.

10. Method according to claim 3, 4, 5, 8 or 9, **characterised in that** (ZO-) is at the para-position and Z represents a group R₄, with n equal to 2.

11. Method according to claim 10, **characterised in that** R₁ and R₂ are identical and represent a linear alkyl group, such as the methyl or ethyl radicals.

12. Method according to claim 3, 4, 5, 8 or 9, **characterised in that** (ZO-) is at the meta or para position, and Z represents a linear alkyl radical, such as the methyl radical.

13. The method for preparing, as claimed in claim 1, compounds of formula (I) as defined in claim 1 in which A represents a group CH—X and Z represents a group R₄, comprising the following steps :
a) a compound of formula (II) : 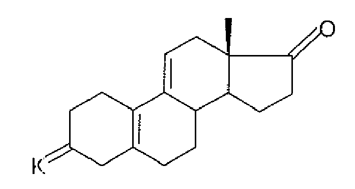 =K representing a keto functional group protected in particular in ketal, thioketal or mixed ketal form,
is subjected to the action of an agent reducing the keto at the 17-position so as to obtain a compound of formula (VII) : 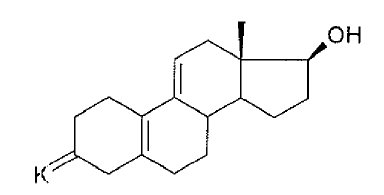
b) the compound of formula (VII) is treated with a halogenating agent so as to obtain a compound of formula (VIII) : 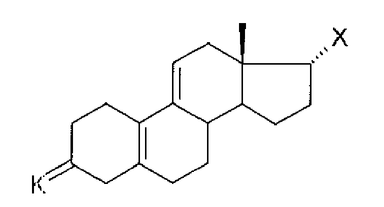 in which X represents a halogen atom,
c) the compound of formula (VIII) is subjected to the action of an epoxidation reagent so as to obtain the mixture of the compounds of formulae (III"a) and (III"b) : 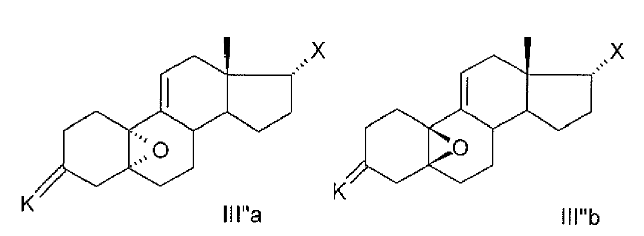
d) the compounds of formulae (III"a) and (III"b) are subjected to an alkylation reaction with an organocuprate derivative derived from an organometallic compound of formula R₅MgHal or R₅Li, Hal being a halogen atom and R₅ representing the group : 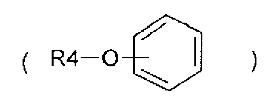 R₄ being as defined in claim 1, the bonding taking place on the phenyl,
and then to the action of a deprotecting agent so as to obtain the compounds of formulae (V " a), (V" b) and (V''c): 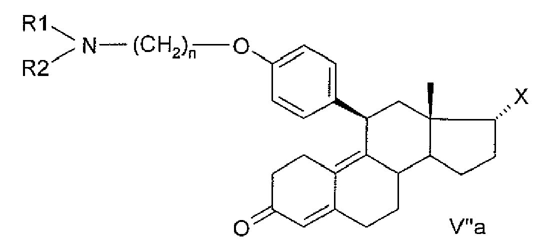 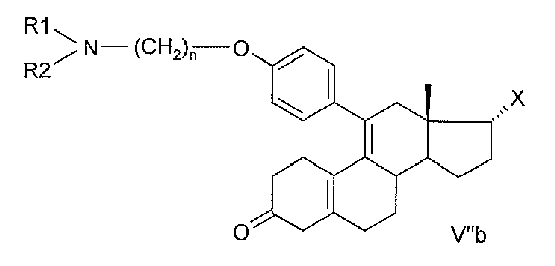 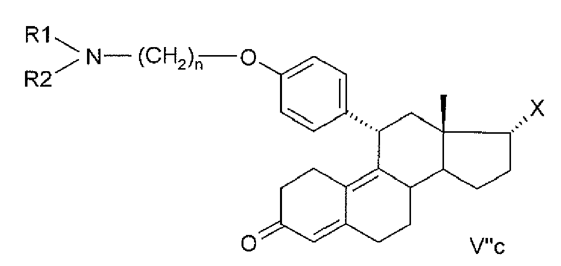
e) the compounds of formulae (V"a), (V"b) and (V"c) are treated with an aromatisation agent so as to obtain the mixture of the compounds of formulae (VI") and (I) in which A represents a group CH-X and Z represents a group R₄, 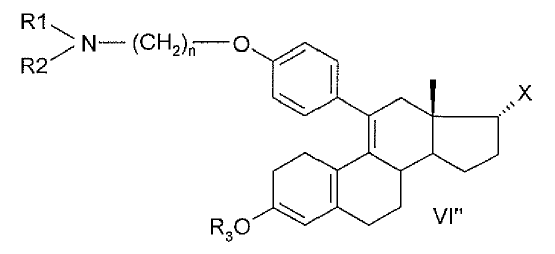 which continue to undergo aromatisation so as to obtain the compound of formula (I) as defined above,
f) where appropriate, the product obtained in step e is deprotected so as to obtain a compound of formula (I) in which A represents a group CH-X, Z represents a group R₄, and R₃ represents a hydrogen atom,
which is subjected, where appropriate, to salification and neutralisation.

14. Method according to claim 13, **characterised in that** =K represents a keto functional group protected in cyclic ketal form, such as 3,3-ethylenedioxy.

15. Method according to claim 13 or 14, **characterised in that** X represents a fluorine atom.

16. Method according to claim 13, 14 or 15, **characterised in that** R₁ and R₂ form together with the nitrogen atom to which they are attached a group 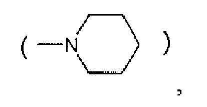 and n is equal to 2.

17. Method according to any one of claims 1 to 16, **characterised in that** the aromatisation agent is acetyl bromide in the presence of acetic anhydride.

18. Method according to any one of claims 1 to 12, **characterised in that** the deprotecting agent used to obtain the compounds of formulae (V'a), (V'c), or (V'b) is an agent allowing acid hydrolysis, such as hydrochloric acid or perchloric acid.

19. As novel intermediate products,
- the compound of general formula (IV'b) as defined in claim 6 and in which Ra, Rb and Rc are identical and represent a methyl,
- the compound of general formula (VI') as defined in claim 3 and in which R₃ represents an acyl group.

20. As novel intermediate products
- the compound of general formula (IV'b) as defined in claim 19 in which (ZO-) is at the para position,
- the compound of general formula (VI') as defined in claim 19 in which (ZO-) is at the para position.

21. As novel intermediate compounds,
- the compound of general formula (VIII) in which =K represents 3,3-ethylenedioxy, and X represents a fluorine atom,
- the compounds of general formulae (III" a) and (III" b) in which =K represents 3,3-ethylenedioxy, and X represents a fluorine atom,
- the compounds of general formulae (V"a), (V"b) and (V"c) in which X represents a fluorine atom, n is equal to 2, and R₁ and R₂ form together with the nitrogen atom to which they are attached a group 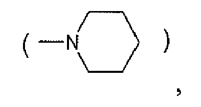
- the compound of general formula (VI") in which R₃ represents an acyl group, X represents a fluorine atom, n is equal to 2, and R₁ and R₂ form together with the nitrogen atom to which they are attached a group 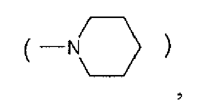
- the compound of general formula (I) in which R₃ represents an acyl group, X represents a fluorine atom, n is equal to 2, and R₁ and R₂ form together with the nitrogen atom to which they are attached a group 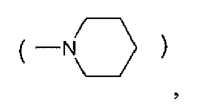 as defined in claim 13.

## Patentansprüche

1. Verfahren zur Herstellung von Verbindungen der allgemeinen Formel (I), 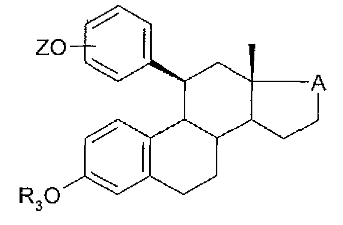 in der
Z einen geradkettigen Alkylrest oder eine Gruppe R₄ bedeutet. R₄ bedeutet: 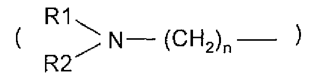 wobei n eine ganze Zahl von 2 bis 8 ist, und
- R₁ und R₂, die gleich oder verschieden sind, eine Benzylgruppe oder einen geradkettigen, verzweigten oder cyclischen Alkyl-, Alkenyl- oder Alkinylrest mit 1 bis 8 Kohlenstoffatomen bedeuten,
- oder R₁ und R₂ gemeinsam mit dem Stickstoffatom, das sie trägt, einen gesättigten oder ungesättigten, aromatischen oder nichtaromatischen Heterocyclus mit 5 bis 6 Ringgliedern, der gegebenenfalls 1 bis 3 zusätzliche Heteroatome enthält und gegebenenfalls mit einem anderen Cyclus verbunden ist, bilden,
A eine Ketofunktion oder eine Gruppe CH-X bedeutet, wobei X ein Halogenatom bedeutet,
R₃ ein Wasserstoffatom oder eine Schutzgruppe der Hydroxyfunktion bedeutet,
das die folgenden Schritte umfasst:
a) man alkyliert die Mischung der Verbindungen der Formeln (lIla) und (IIIb): 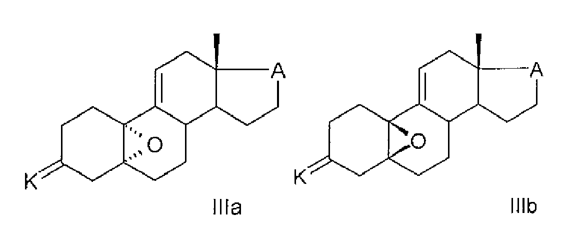 in der =K eine insbesondere in Form eines Ketals, Thioketals, oder Mischketals geschützte Ketofunktion bedeutet,
mit einem kupferorganischen Derivat, das sich von einer metallorganischen Verbindung der Formel R₅MgHal oder R₅Li, wobei Hal ein Halogenatom bedeutet, ableitet und das katalytisch oder stöchiometrisch hergestellt worden ist, wobei R₅ die Gruppe 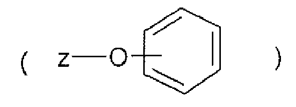 bedeutet, wobei Z wie oben definiert ist, wobei die Bindung am Phenyl erfolgt,
wonach das Produkt mit einem Entschützungsmittel behandelt wird,
wodurch man die Verbindungen der Formeln (Va), (Vb) und (Vc) 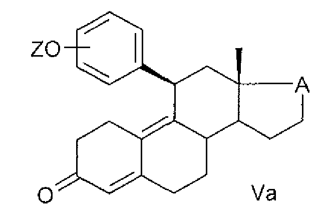 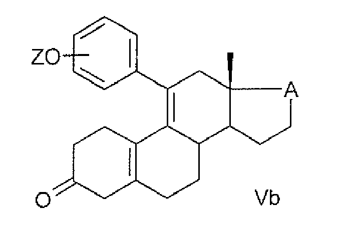 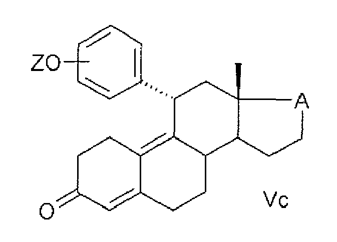 erhält;
b) man behandelt die Verbindungen der Formeln (Va), (Vb) und (Vc) mit einem Aromatisierungsmittel, wodurch man die Mischung der Verbindungen der Formeln (VI) und (I) erhält, 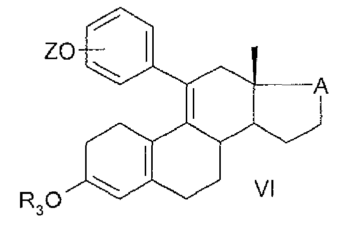 die weiter zu der Verbindung der Formel (I) aromatisieren, wobei R₃ wie oben definiert ist.

2. Verfahren zur Herstellung von Verbindungen der allgemeinen Formel (I), 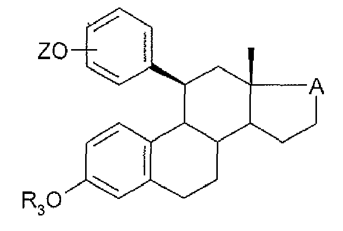 in der
Z einen geradkettigen Alkylrest oder eine Gruppe R₄ bedeutet. R₄ bedeutet: 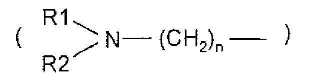 wobei n eine ganze Zahl von 2 bis 8 ist, und
- R₁ und R₂ , die gleich oder verschieden sind, eine Benzylgruppe oder einen geradkettigen, verzweigten oder cyclischen Alkyl-, Alkenyl- oder Alkinylrest mit 1 bis 8 Kohlenstoffatomen bedeuten,
- oder R₁ und R₂ gemeinsam mit dem Stickstoffatom, das sie trägt, einen gesättigten oder ungesättigten, aromatischen oder nichtaromatischen Heterocyclus mit 5 bis 6 Ringgliedern, der gegebenenfalls 1 bis 3 zusätzliche Heteroatome enthält und gegebenenfalls mit einem anderen Cyclus verbunden ist, bilden,
A eine Ketofunktion oder eine Gruppe CH-X bedeutet, wobei X ein Halogenatom bedeutet,
R₃ ein Wasserstoffatom oder eine Schutzgruppe der Hydroxyfunktion bedeutet,
das die folgenden Schritte umfasst:
a)man alkyliert die Verbindung der Formel (IIIb) 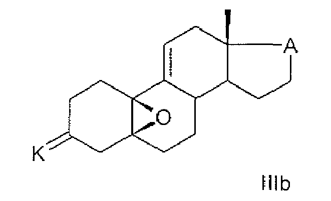 in der =K eine insbesondere in Form eines Ketals, Thioketals, oder Mischketals geschützte Ketofunktion bedeutet,
mit einem kupferorganischen Derivat, das sich von einer metallorganischen Verbindung der Formel R₅MgHal oder R₅Li, wobei Hal ein Halogenatom bedeutet, ableitet und das katalytisch oder stöchiometrisch hergestellt worden ist, wobei R₅ die Gruppe 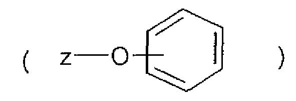 bedeutet, wobei Z wie oben definiert ist, wobei die Bindung am Phenyl erfolgt,
wonach das Produkt mit einem Entschützungsmittel behandelt wird,
wodurch man die Verbindungen der Formeln (Vb) und (Vc) 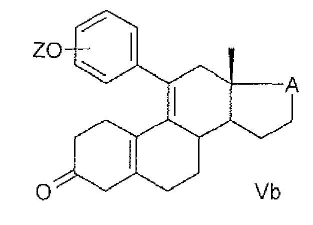 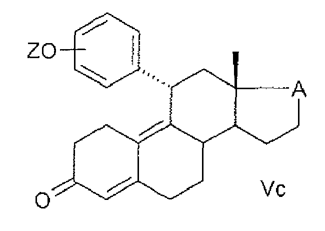 erhält;
b)man behandelt die Verbindungen der Formeln (Vb) und (Vc) mit einem Aromatisierungsmittel, wodurch man die Mischung der Verbindungen der Formeln (VI) und (I) erhält, 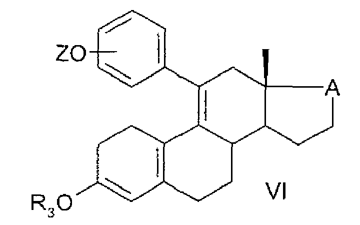 die weiter zu der Verbindung der Formel (I) aromatisieren, wobei R₃ wie oben definiert ist.

3. Verfahren nach Anspruch 1 zur Herstellung von Verbindungen der allgemeinen Formel (I), in der A eine Ketofunktion bedeutet, das die folgenden Schritte umfasst:
a) man behandelt eine Verbindung der Formel (II), 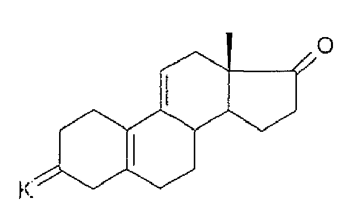 in der =K eine insbesondere in Form eines Ketals, Thioketals, oder Mischketals geschützte Ketofunktion bedeutet,
mit einem Epoxidationsmittel, wodurch man zu einer Mischung der alpha- und beta-Isomere der Formeln (III'a) und (III'b) gelangt; 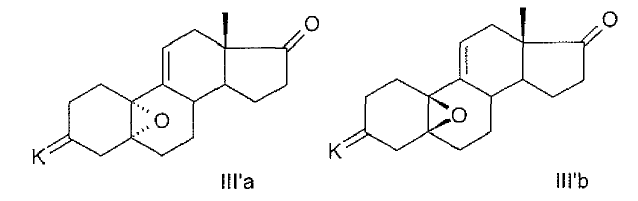
b) man alkyliert die Mischung der Verbindungen der Formeln (III'a) und (III'b) mit einem kupferorganischen Derivat, das von einer metallorganischen Verbindung der Formel R₅MgHal oder R₅Li abgeleitet ist, wobei Hal ein Halogenatom bedeutet und R₅ wie in Anspruch 1 definiert ist, wobei die Bindung am Phenyl erfolgt,
und behandelt das Produkt anschließend mit einem Entschützungsmittel, wodurch man zu den Verbindungen der Formeln (V'a), (V'b) und (V'c) gelangt: 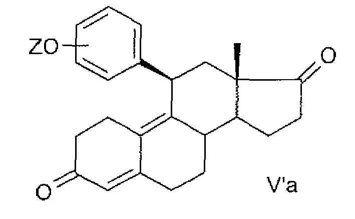 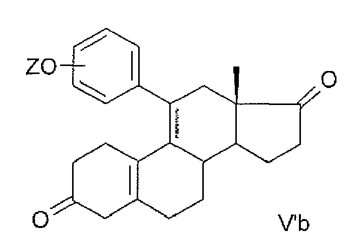 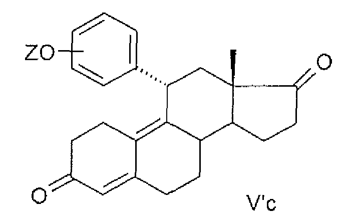
c) man behandelt die Verbindungen der Formeln (V'a), (V'b) und (V'c) mit einem Aromatisierungsmittel, wodurch man zu einer Mischung der Verbindungen der Formeln (VI') und (I) gelangt, in der A eine Ketofunktion bedeutet: 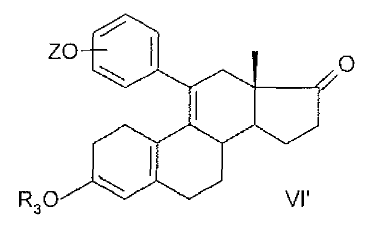 die weiter zu der Verbindung der Formel (I) aromatisieren, in der A eine Ketofunktion bedeutet,
d) gegebenenfalls Entschützen des in Stufe c erhaltenen Produkts, wodurch man zu einer Verbindung der Formel (I) gelangt, in der A eine Ketofunktion bedeutet und R₃ ein Wasserstoffatom bedeutet,
und, gegebenenfalls, Durchführen einer Salzbildungsreaktion.

4. Verfahren nach Anspruch 2 zur Herstellung von Verbindungen der allgemeinen Formel (I), in der A eine Ketofunktion bedeutet, das die folgenden Schritte umfasst:
a) man behandelt eine Verbindung der Formel (II), 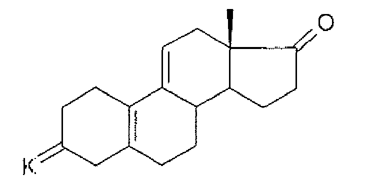 in der =K eine insbesondere in Form eines Ketals, Thioketals, oder Mischketals geschützte Ketofunktion bedeutet,
mit einem Epoxidationsmittel, wodurch man das beta-Isomer der Formel (III'b) erhält: 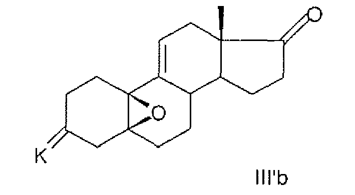
b) man alkyliert die Verbindung der Formel (III'b) mit einem kupferorganischen Derivat, das von einer metallorganischen Verbindung der Formel R₅MgHal oder R₅Li abgeleitet ist, wobei Hal ein Halogenatom bedeutet und R₅ wie in Anspruch 2 definiert ist, wobei die Bindung am Phenyl erfolgt,
und behandelt das Produkt anschließend mit einem Entschützungsmittel, wodurch man zu den Verbindungen der Formeln (V'b) und (V'c) gelangt: 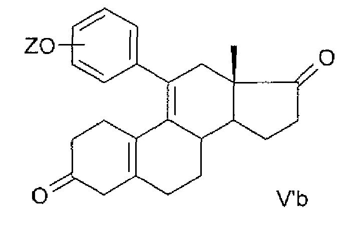 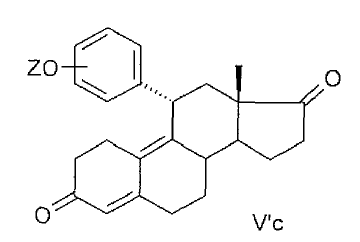
c) man behandelt die Verbindungen der Formeln (V'b) und (V'c) mit einem Aromatisierungsmittel, wodurch man zu einer Mischung der Verbindungen der Formeln (VI') und (I) gelangt, in der A eine Ketofunktion bedeutet: 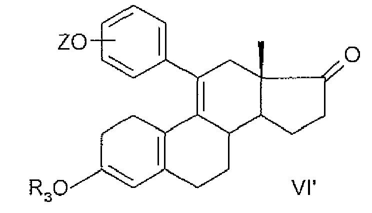 die weiter zu der Verbindung der Formel (I) aromatisieren, in der A eine Ketofunktion bedeutet,
d) gegebenenfalls Entschützen des in Stufe c erhaltenen Produkts, wodurch man zu einer Verbindung der Formel (I) gelangt, in der A eine Ketofunktion bedeutet und R₃ ein Wasserstoffatom bedeutet,
und, gegebenenfalls, Durchführen einer Salzbildungsreaktion.

5. Verfahren nach Anspruch 3 oder 4, **dadurch gekennzeichnet, dass** die Alkylierung von einer Enolisierung begleitet ist.

6. Verfahren nach Anspruch 3, **dadurch gekennzeichnet, dass** man die Verbindungen der Formeln (III'a) und (III'b)
in Gegenwart einer Base mit einem Silylierungsmittel behandelt, wodurch man eine Mischung von silylierten Enolen der Formeln (IVa) und (IVb) erhält: 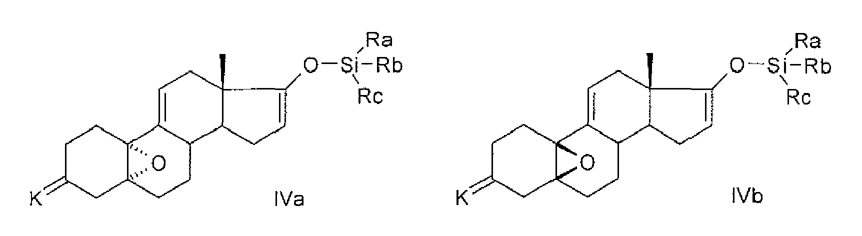 in denen Ra, Rb und Rc, die gleich oder verschieden sind, einen Alkylrest mit 1 bis 4 Kohlenstoffatomen oder einen Phenylrest bedeuten,
die man mit einem kupferorganischen Derivat wie in Anspruch 3 definiert umsetzt, wobei die Bindung am Phenyl erfolgt,
wodurch man zu den Verbindungen der Formeln (IV'a) und (IV'b) gelangt, die gegebenenfalls isoliert werden: 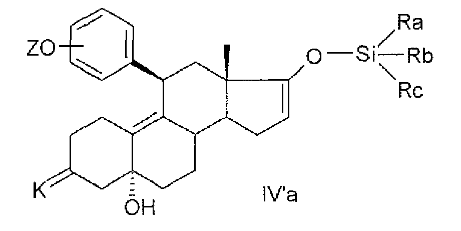 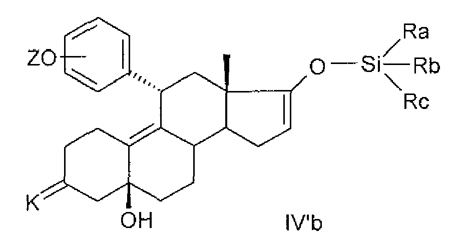 wobei diese Produkte dann entschützt werden, wodurch man zu den Verbindungen der Formeln (V'a), (V'b), (V'c) wie in Anspruch 3 definiert gelangt.

7. Verfahren nach Anspruch 4, **dadurch gekennzeichnet, dass** man die Verbindung der Formel (III'b) in Gegenwart einer Base mit einem Silylierungsmittel behandelt, wodurch man zu dem silylierten Enol der Formel (IVb) gelangt, 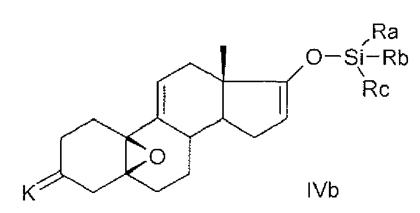 in der Ra, Rb und Rc, die gleich oder verschieden sind, einen Alkylrest mit 1 bis 4 Kohlenstoffatomen oder einen Phenylrest bedeuten,
die man mit einem kupferorganischen Derivat wie in Anspruch 4 definiert umsetzt, wobei die Bindung am Phenyl erfolgt,
wodurch man zu der Verbindung der Formel (IV'b) gelangt, die gegebenenfalls isoliert wird: 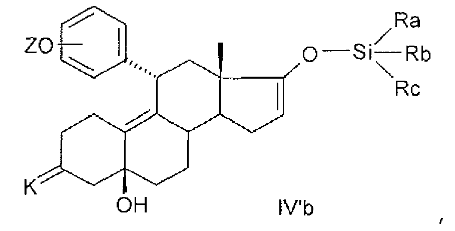 wobei das Produkt anschließend entschützt wird, wodurch man zu den Verbindungen der Formeln (V'b), (V'c) wie in Anspruch 4 definiert gelangt.

8. Verfahren nach Anspruch 6 oder 7, **dadurch gekennzeichnet, dass** es sich bei dem Silylderivat um ein Trimethylsilylderivat handelt, mit dem man die silylierten Enole der Formeln (IVb) und/oder (IVa) erhält, in denen Ra, Rb und Rc gleich oder verschieden sind und ein Methyl bedeuten.

9. Verfahren nach Anspruch 3, 4, 5 oder 8, **dadurch gekennzeichnet, dass** =K eine in Form eines cyclischen Ketals geschützte Ketofunktion, wie 3,3-Ethylendioxy, bedeutet.

10. Verfahren nach Anspruch 3, 4, 5, 8 oder 9, **dadurch gekennzeichnet, dass** (ZO-) in para-Stellung steht und Z eine Gruppe R₄ bedeutet, wobei n gleich 2 ist.

11. Verfahren nach Anspruch 10, **dadurch gekennzeichnet, dass** R₁ und R₂ identisch sind und eine geradkettige Alkylgruppe, wie den Methylrest oder den Ethylrest, bedeuten.

12. Verfahren nach Anspruch 3, 4, 5, 8 oder 9, **dadurch gekennzeichnet, dass** (ZO-) in meta- oder para-Stellung steht und Z einen geradkettigen Alkylrest, wie den Methylrest, bedeutet.

13. Verfahren nach Anspruch 1 zur Herstellung von Verbindungen der Formel (I) wie in Anspruch 1 definiert, in der A eine Gruppe CH-X bedeutet und Z eine Gruppe R₄ bedeutet, das die folgenden Schritte umfasst:
a) man behandelt eine Verbindung der Formel (II) 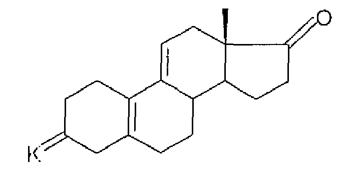 in der =K eine insbesondere in Form eines Ketals, Thioketals oder Mischketals geschützte Ketofunktion bedeutet,
mit einem 17-Ketoreduktionsmittel, wodurch man zu einer Verbindung der Formel (VII) gelangt; 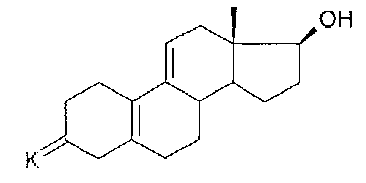
b) man behandelt die Verbindung der Formel (VII) mit einem Halogenierungsmittel, wodurch man zu einer Verbindung der Formel (VIII) gelangt, 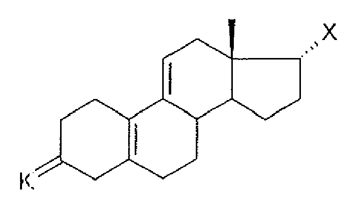 in der X ein Halogenatom bedeutet;
c) man behandelt die Verbindung der Formel (VIII) mit einem Epoxidationsmittel, wodurch man zu einer Mischung der Verbindungen der Formeln (III"a) und (III"b) gelangt: 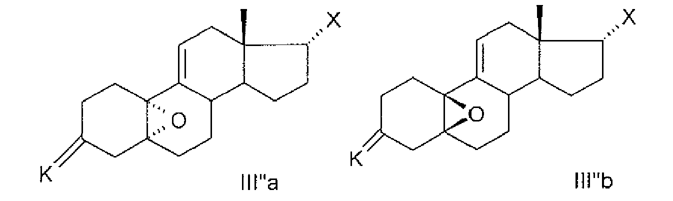
d) man alkyliert die Verbindungen der Formeln (III"a) und (III"b) mit einem kupferorganischen Derivat, das von einer metallorganischen Verbindung der Formel R₅MgHal oder R₅Li abgeleitet ist, wobei Hal ein Halogenatom bedeutet und R₅ die Gruppe 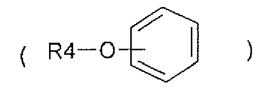 bedeutet, wobei R₄ wie in Anspruch 1 definiert ist, und wobei die Bindung am Phenyl erfolgt,
und anschließend mit einem Entschützungsmittel, wodurch man zu den Verbindungen der Formeln (V"a), (V"b) und (V"c) gelangt: 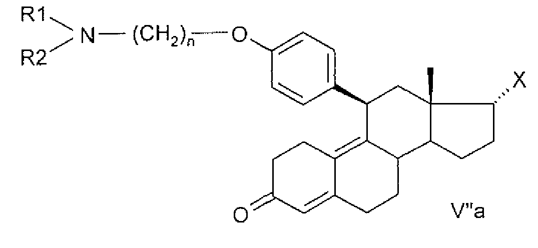 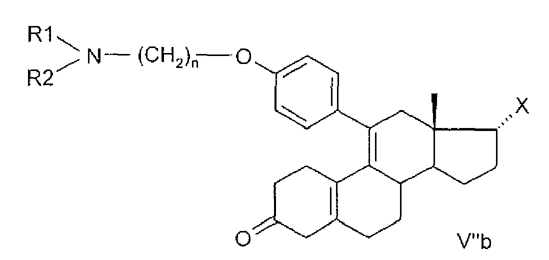 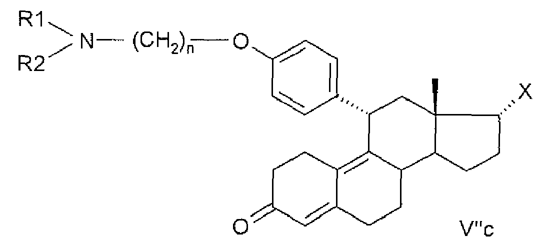
e) man behandelt die Verbindungen der Formeln (V''a), (V"b) und (V"c) mit einem Aromatisierungsmittel, wodurch man zu einer Mischung der Verbindungen der Formeln (VI'') und (I), worin A eine Gruppe CH-X bedeutet und Z eine Gruppe R₄ bedeutet, gelangt, 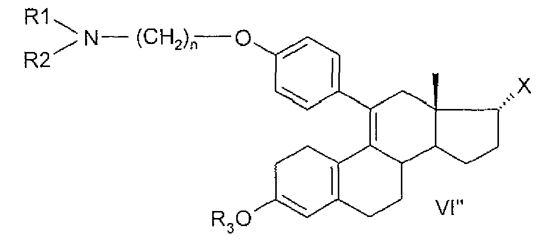 die weiter zu der Verbindung der Formel (I) wie oben definiert aromatisieren;
f) gegebenenfalls Entschützen des in Stufe e erhaltenen Produkts, wodurch man zu einer Verbindung der Formel (I) gelangt, in der A eine Gruppe CH-X bedeutet, Z eine Gruppe R₄ bedeutet und R₃ ein Wasserstoffatom bedeutet,
mit der gegebenenfalls eine Salzbildung und eine Neutralisierung durchgeführt wird.

14. Verfahren nach Anspruch 13, **dadurch gekennzeichnet, dass** =K eine insbesondere in Form eines cyclischen Ketals, wie 3,3-Ethylendioxy, geschützte Ketofunktion bedeutet.

15. Verfahren nach Anspruch 13 oder 14, **dadurch gekennzeichnet, dass** X ein Fluoratom bedeutet.

16. Verfahren nach Anspruch 13, 14 oder 15, **dadurch gekennzeichnet, dass** R₁ und R₂ gemeinsam mit dem Stickstoffatom, an das sie gebunden sind, eine Gruppe 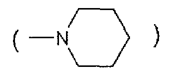 bilden und n gleich 2 ist.

17. Verfahren nach einem der Ansprüche 1 bis 16, **dadurch gekennzeichnet, dass** es sich bei dem Aromatisierungsmittel um Acetylbromid in Gegenwart von Essigsäureanhydrid handelt.

18. Verfahren nach einem der Ansprüche 1 bis 12, **dadurch gekennzeichnet, dass** es sich bei dem zwecks Gewinnung der Verbindungen der Formeln (V'a), (V'c) oder (V'b) verwendeten Entschützungsmittel um ein Mittel, das eine saure Hydrolyse gestattet, handelt, wie Chlorwasserstoffsäure oder Perchlorsäure.

19. Als neue Zwischenprodukte,
- die Verbindung der allgemeinen Formel (IV'b) wie in Anspruch 6 definiert, in der Ra, Rb und Rc gleich sind und Methyl bedeuten,
- die Verbindung der allgemeinen Formel (VI'), wie in Anspruch 3 definiert, in der R₃ eine Acylgruppe bedeutet.

20. Als neue Zwischenprodukte,
- die Verbindung der allgemeinen Formel (IV'b) wie in Anspruch 19 definiert, in der (ZO-) in para-Stellung steht,
- die Verbindung der allgemeinen Formel (VI') wie in Anspruch 19 definiert, in der (ZO-) in para-Stellung steht.

21. Als neue Zwischenprodukte,
- die Verbindung der allgemeinen Formel (VIII), in der =K 3,3-Ethylendioxy bedeutet und X ein Fluoratom bedeutet,
- die Verbindungen der allgemeinen Formeln (III"a) und (III" b), in denen =K 3,3-Ethylendioxy bedeutet und X ein Fluoratom bedeutet,
- die Verbindungen der allgemeinen Formeln (V"a), (V"b) und (V"c), in denen X ein Fluoratom bedeutet, n gleich 2 ist und R₁ und R₂ gemeinsam mit dem Stickstoffatom an das sie gebunden sind, eine Gruppe 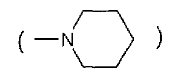 bilden,
- die Verbindung der allgemeinen Formel (VI''), in der R₃ eine Acylgruppe bedeutet, X ein Fluoratom bedeutet, n gleich 2 ist und R₁ und R₂ gemeinsam mit dem Stickstoffatom, an das sie gebunden sind, eine Gruppe 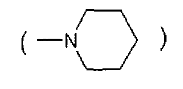 bilden,
- die Verbindung der allgemeinen Formel (I), in der R₃ eine Acylgruppe bedeutet, X ein Fluoratom bedeutet, n gleich 2 ist und R₁ und R₂ gemeinsam mit dem Stickstoffatom, an das sie gebunden sind, eine Gruppe 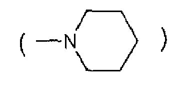 bilden, wie sie in Anspruch 13 definiert sind.
